# EUROPEAN PATENT APPLICATION

(11) **EP 1 897 954 A1**
(43) Date of publication of application: **12.03.2008**
(21) Application number: 06076710.0
(22) Date of filing: 11.09.2006
(51) Int. Cl.: C12N 15/82, A01H 5/00

(54) **Methods and means for producing starch having at least one altered characteristic**

(71) Applicant: Wageningen Universiteit, 6701 BH Wageningen (NL)
(72) Inventor: Vincken, Jean-Paul, 6871 DH Renkum (NL); Nazarian Firouzabadi, Farhad, 6708 DM Wageningen (NL); Visser, Richard Gerardus Franciscus, 6721 ET Bennekom (NL)
(74) Representative: van Loon, C.J.J.

(57) **Abstract**

The invention provides a method for modifying a starch producing plant and a method for producing starch having at least one altered characteristic. The invention particularly provides a method for producing starch comprising introducing a nucleic acid comprising an amylosucrase encoding sequence functionally linked to a granule-targeting sequence and functionally linked to a promoter sequence active in a starch storage organ of said plant into suitable plant material of said plant and producing a plant from said material, further comprising allowing said plant to grow and harvesting starch from said plant. The invention further provides starch having an altered characteristic and plants and parts thereof and products comprising such starch. Furthermore, the invention provides fusion proteins and cells comprising a fusion protein.

SEQUENCE LISTING

<110>  Wageningen Universiteit
       VINCKEN, Jean-Paul
       NAZARIAN FIROUZABADI, Farhad
       VISSER, Richard Gerardus Franciscus

<120>  Methods and means for producing starch having at least one
       alterered characteristic

<130>  P77257EP00

<140>  EP 06076710.0
<141>  2006-09-11

<160>  15

<170>  PatentIn version 3.3

<210>  1
<211>  19
<212>  DNA
<213>  Artificial

<220>
<223>  Primer 1

<400>  1
cgaaaaggat cccccgaat                                                    19


<210>  2
<211>  19
<212>  DNA
<213>  Artificial

<220>
<223>  Primer 2

<400>  2
tctctgcagc gccaaaaca                                                    19


<210>  3
<211>  21
<212>  DNA
<213>  Artificial

<220>
<223>  Primer 3

<400>  3
tgacgaaaag gatccattgt c                                                 21


<210>  4
<211>  19
<212>  DNA
<213>  Artificial

<220>
<223>  Primer 4

<400>  4
tactgcaggc atttgggaa                                                    19


<210>  5

<211>  106
<212>  PRT
<213>  Bacillus circulans


<220>
<221>  BINDING
<222>  (1)..(106)
<223>  Starch binding domain

<400>  5

Ser Gly Asp Gln Val Ser Val Arg Phe Val Val Asn Asn Ala Thr Thr
1               5                   10                  15


Ala Leu Gly Gln Asn Val Tyr Leu Thr Gly Ser Val Ser Glu Leu Gly
            20                  25                  30


Asn Trp Asp Pro Ala Lys Ala Ile Gly Pro Met Tyr Asn Gln Val Val
        35                  40                  45


Tyr Gln Tyr Pro Asn Trp Tyr Tyr Asp Val Ser Val Pro Ala Gly Lys
    50                  55                  60


Thr Ile Glu Phe Lys Phe Leu Lys Lys Gln Gly Ser Thr Val Thr Trp
65                  70                  75                  80


Glu Gly Gly Ser Asn His Thr Phe Thr Ala Pro Ser Ser Gly Thr Ala
                85                  90                  95


Thr Ile Asn Val Asn Trp Gln Arg Ser Pro
            100                 105


<210>  6
<211>  108
<212>  PRT
<213>  Aspergillus niger


<220>
<221>  BINDING
<222>  (1)..(107)
<223>  Starch binding domain

<400>  6

<210>  7
<211>  104
<212>  PRT
<213>  Clostridium thermosulfurigenes

<220>
<221>  BINDING
<222>  (1)..(104)
<223>  Starch binding domain

<400>  7

Thr Gly Asn Gln Ile Cys Val Arg Phe Val Val Asn Asn Ala Ser Thr
1               5                   10                  15

Val Tyr Gly Glu Asn Val Tyr Leu Thr Gly Asn Val Ala Glu Leu Gly
            20                  25                  30

Asn Trp Asp Thr Ser Lys Ala Ile Gly Pro Met Phe Asn Gln Val Val
        35                  40                  45

Tyr Gln Tyr Pro Thr Trp Tyr Tyr Asp Val Ser Val Pro Ala Gly Thr
    50                  55                  60

Thr Ile Gln Phe Lys Phe Ile Lys Lys Asn Gly Asn Thr Ile Thr Trp
65                  70                  75                  80

Glu Gly Gly Ser Asn His Thr Tyr Thr Val Pro Ser Ser Ser Thr Gly
                85                  90                  95

Thr Val Ile Val Asn Trp Gln Gln
            100

<210>  8
<211>  106
<212>  PRT
<213>  Bacillus stearothermophilus

<220>
<221>  BINDING
<222>  (1)..(106)
<223>  Starch binding domain

<400>  8

Thr Asn Asp Gln Val Ser Val Arg Phe Val Val Asn Asn Ala Thr Thr

1               5                   10                  15

Asn Leu Gly Gln Asn Ile Tyr Ile Val Gly Asn Val Tyr Glu Leu Gly
            20                  25                  30

Asn Trp Asp Thr Ser Lys Ala Ile Gly Pro Met Glu Asn Gln Val Val
        35                  40                  45

Tyr Ser Tyr Pro Thr Trp Tyr Ile Asp Val Ser Val Pro Glu Gly Lys
    50                  55                  60

Thr Ile Glu Phe Lys Phe Ile Lys Lys Asp Ser Gln Gly Asn Asn Val
65                  70                  75                  80

Thr Trp Glu Ser Gly Ser Asn His Val Tyr Thr Thr Pro Thr Asn Thr
                85                  90                  95

Thr Gly Lys Ile Ile Val Asp Trp Gln Asn
            100                 105

<210>  9
<211>  566
<212>  PRT
<213>  Neisseria polysaccharea

<400>  9

Gln Tyr Leu Lys Thr Arg Ile Leu Asp Ile Tyr Thr Pro Glu Gln Arg
1               5                   10                  15

Ala Gly Ile Glu Lys Ser Glu Asp Trp Arg Gln Phe Ser Arg Arg Met
            20                  25                  30

Asp Thr His Phe Pro Lys Leu Met Asn Glu Leu Asp Ser Val Tyr Gly
        35                  40                  45

Asn Asn Glu Ala Leu Leu Pro Met Leu Glu Met Leu Leu Ala Gln Ala
    50                  55                  60

Trp Gln Ser Tyr Ser Gln Arg Asn Ser Ser Leu Lys Asp Ile Asp Ile
65                  70                  75                  80

Ala Arg Glu Asn Asn Pro Asp Trp Ile Leu Ser Asn Lys Gln Val Gly
                85                  90                  95

Gly Val Cys Tyr Val Asp Leu Phe Ala Gly Asp Leu Lys Gly Leu Lys
            100                 105                 110

Asp Lys Ile Pro Tyr Phe Gln Glu Leu Gly Leu Thr Tyr Leu His Leu
        115                 120                 125

Met Pro Leu Phe Lys Cys Pro Glu Gly Lys Ser Asp Gly Gly Tyr Ala
    130                 135                 140

Val Ser Ser Tyr Arg Asp Val Asn Pro Ala Leu Gly Thr Ile Gly Asp
145                 150                 155                 160

Leu Arg Glu Val Ile Ala Ala Leu His Glu Ala Gly Ile Ser Ala Val
                165                 170                 175

Val Asp Phe Ile Phe Asn His Thr Ser Asn Glu His Glu Trp Ala Gln
            180                 185                 190

Arg Cys Ala Ala Gly Asp Pro Leu Phe Asp Asn Phe Tyr Tyr Ile Phe
        195                 200                 205

Pro Asp Arg Arg Met Pro Asp Gln Tyr Asp Arg Thr Leu Arg Glu Ile
    210                 215                 220

Phe Pro Asp Gln His Pro Gly Gly Phe Ser Gln Leu Glu Asp Gly Arg
225                 230                 235                 240

Trp Val Trp Thr Thr Phe Asn Ser Phe Gln Trp Asp Leu Asn Tyr Ser
                245                 250                 255

Asn Pro Trp Val Phe Arg Ala Met Ala Gly Glu Met Leu Phe Leu Ala
            260                 265                 270

Asn Leu Gly Val Asp Ile Leu Arg Met Asp Ala Val Ala Phe Ile Trp
        275                 280                 285

Lys Gln Met Gly Thr Ser Cys Glu Asn Leu Pro Gln Ala His Ala Leu
    290                 295                 300

Ile Arg Ala Phe Asn Ala Val Met Arg Ile Ala Ala Pro Ala Val Phe
305                 310                 315                 320

Phe Lys Ser Glu Ala Ile Val His Pro Asp Gln Val Val Gln Tyr Ile
                325                 330                 335

Gly Gln Asp Glu Cys Gln Ile Gly Tyr Asn Pro Leu Gln Met Ala Leu
            340                 345                 350

Leu Trp Asn Thr Leu Ala Thr Arg Glu Val Asn Leu Leu His Gln Ala
        355                 360                 365

Leu Thr Tyr Arg His Asn Leu Pro Glu His Thr Ala Trp Val Asn Tyr
    370                 375                 380

Val Arg Ser His Asp Asp Ile Gly Trp Thr Phe Ala Asp Glu Asp Ala
385                 390                 395                 400

Ala Tyr Leu Gly Ile Ser Gly Tyr Asp His Arg Gln Phe Leu Asn Arg
                405                 410                 415

Phe Phe Val Asn Arg Phe Asp Gly Ser Phe Ala Arg Gly Val Pro Phe
            420                 425                 430

Gln Tyr Asn Pro Ser Thr Gly Asp Cys Arg Val Ser Gly Thr Ala Ala
        435                 440                 445

Ala Leu Val Gly Leu Ala Gln Asp Asp Pro His Ala Val Asp Arg Ile
    450                 455                 460

Lys Leu Leu Tyr Ser Ile Ala Leu Ser Thr Gly Gly Leu Pro Leu Ile
465                 470                 475                 480

Tyr Leu Gly Asp Glu Val Gly Thr Leu Asn Asp Asp Asp Trp Ser Gln
                485                 490                 495

Asp Ser Asn Lys Ser Asp Asp Ser Arg Trp Ala His Arg Pro Arg Tyr
            500                 505                 510

Asn Glu Ala Leu Tyr Ala Gln Arg Asn Asp Pro Ser Thr Ala Ala Gly
        515                 520                 525

Gln Ile Tyr Gln Gly Leu Arg His Met Ile Ala Val Arg Gln Ser Asn
    530                 535                 540

Pro Arg Phe Asp Gly Gly Arg Leu Val Thr Phe Asn Thr Asn Asn Lys
545                 550                 555                 560

His Ile Ile Gly Tyr Ile
                565

<210>  10
<211>  636
<212>  PRT
<213>  Neisseria meningitidis

<400>  10

Met Leu Thr Pro Thr Gln Gln Val Gly Leu Ile Leu Gln Tyr Leu Lys
1               5                   10                  15

Thr Arg Ile Leu Asp Ile Tyr Thr Pro Glu Gln Arg Ala Gly Ile Glu
            20                  25                  30

Lys Ser Glu Asp Trp Arg Gln Phe Ser Arg Arg Met Asp Thr His Phe
        35                  40                  45

Pro Lys Leu Met Asn Glu Leu Asp Ser Val Tyr Gly Asn Asn Glu Ala
    50                  55                  60

Leu Leu Pro Met Leu Glu Met Leu Leu Ala Gln Ala Trp Gln Ser Tyr
65                  70                  75                  80

Ser Gln Arg Ser Ala Ser Leu Lys Asn Ile Asp Ile Glu Arg Glu Asn
                85                  90                  95

Asn Pro Asp Trp Ile Leu Ser Asn Lys Gln Val Gly Gly Val Cys Tyr
            100                 105                 110

Val Asp Leu Phe Ala Gly Asp Leu Lys Gly Leu Lys Asp Lys Ile His
        115                 120                 125

Tyr Phe Gln Glu Leu Gly Leu Thr Tyr Leu His Leu Met Pro Leu Phe
    130                 135                 140

Lys Cys Pro Glu Gly Lys Ser Asp Gly Gly Tyr Ala Val Ser Ser Tyr
145                 150                 155                 160

Arg Asp Val Asn Pro Ala Leu Gly Thr Ile Gly Asp Leu Arg Glu Val
                165                 170                 175

Ile Ala Ala Leu His Glu Ala Gly Ile Ser Ala Val Val Asp Phe Ile
            180                 185                 190

Phe Asn His Thr Ser Asn Glu His Glu Trp Ala Gln Arg Cys Ala Ala
        195                 200                 205

Gly Asp Pro Leu Phe Asp Asn Phe Tyr Tyr Ile Phe Pro Asp Arg Arg
    210                 215                 220

Met Pro Asp Gln Tyr Asp Arg Thr Leu Arg Glu Ile Phe Pro Asp Gln
225                 230                 235                 240

His Pro Gly Gly Phe Ser Gln Leu Glu Asp Gly Arg Trp Val Trp Thr
                245                 250                 255

Thr Phe Asn Ser Phe Gln Trp Asp Leu Asn Tyr Ser Asn Pro Trp Val
            260                 265                 270

Phe Arg Ala Met Ala Gly Glu Met Met Phe Leu Ala Asn Leu Gly Val
        275                 280                 285

Asp Ile Leu Arg Met Asp Ala Val Ala Phe Ile Trp Lys Gln Met Gly
    290                 295                 300

Thr Ser Cys Glu Asn Leu Pro Gln Ala His Ala Leu Ile Arg Ala Phe
305                 310                 315                 320

Asn Ala Val Met Arg Ile Ala Ala Pro Ala Val Phe Phe Lys Ser Glu
                325                 330                 335

Ala Ile Val His Pro Asp Gln Val Val Gln Tyr Ile Gly Gln Asp Glu
            340                 345                 350

Cys Gln Ile Gly Tyr Asn Pro Leu Gln Met Ala Leu Leu Trp Asn Thr
        355                 360                 365

Leu Ala Thr Arg Glu Val Asn Leu Leu His Gln Ala Leu Thr Tyr Arg
    370                 375                 380

His Asn Leu Pro Glu His Thr Ala Trp Val Asn Tyr Val Arg Ser His
385                 390                 395                 400

Asp Asp Ile Gly Trp Thr Phe Ala Asp Glu Asp Ala Ala Tyr Leu Gly
                405                 410                 415

Ile Ser Gly Tyr Asp His Arg Gln Phe Leu Asn Arg Phe Phe Val Asn
            420                 425                 430

Arg Phe Asp Gly Ser Phe Ala Arg Gly Val Pro Phe Gln Tyr Asn Pro
        435                 440                 445

Asn Thr Gly Asp Cys Arg Val Ser Gly Thr Ser Ala Ala Leu Ala Gly
    450                 455                 460

Leu Ala Gln Asn Asp Pro His Ala Val Ser Arg Ile Lys Leu Leu Tyr
465                 470                 475                 480

Ser Ile Ala Leu Ser Thr Gly Gly Leu Pro Leu Ile Tyr Leu Gly Asp
                485                 490                 495

Glu Val Gly Thr Pro Asn Asp Asp Gly Trp Val Gln Asp Ser Asn Lys
            500                 505                 510

Ser Asp Asp Ser Arg Trp Ala His Arg Pro Arg Tyr Asn Glu Ala Leu
        515                 520                 525

Tyr Ala Gln Arg Asn Asp Pro Ser Thr Ala Ala Gly Gln Ile Tyr Gln
    530                 535                 540

Gly Leu Arg His Met Ile Ala Val Arg Gln Ser Asn Pro Arg Phe Asp
545                 550                 555                 560

Gly Gly Arg Leu Val Thr Phe Asn Thr Asn Asn Lys His Ile Ile Gly
                565                 570                 575

Tyr Ile Arg Asn Asn Ala Leu Leu Ala Phe Gly Asn Phe Ser Glu Tyr
            580                 585                 590

Pro Gln Thr Val Thr Ala His Thr Leu Gln Ala Met Pro Phe Lys Ala
        595                 600                 605

His Asp Leu Ile Gly Gly Lys Thr Val Ser Leu Asn Gln Asp Leu Thr
    610                 615                 620

Leu Gln Pro Tyr Gln Val Met Trp Leu Glu Ile Ala
625                 630                 635

<210>  11
<211>  701
<212>  PRT
<213>  Magentoccoccus sp.

<400>  11

Met Lys Asn Arg Thr Asn Ile Lys Thr Thr Asn Pro Gln Ala Ile Arg
1               5                   10                  15

Met Thr Thr Ala Thr Asn Val Asn Leu Pro Lys Pro Pro Pro Leu Pro
            20                  25                  30

Glu Pro Glu Ser Ala Pro Ala Glu Thr Met Asn Ala Met Leu Thr Asn
        35                  40                  45

Ala Leu Met His Ala Val Arg Asn Gly Val Lys Asp Ser Arg Ala Gln
    50                  55                  60

Ser Ala Leu Asn Arg Leu Leu Pro Lys Leu Glu Ser Phe Trp Glu Val
65                  70                  75                  80

Glu Asn Val Ser Gln Gln Glu Ala Asp Glu Ile Gln Leu Arg Ile Val
                85                  90                  95

Gln His Trp Asp Asp Leu Phe Gly Tyr Leu Leu His Leu Tyr Gly Asp
            100                 105                 110

Gln Trp Asp Phe Phe Tyr His Leu Glu Gln Ile Leu Leu Thr Ile Ile
        115                 120                 125

Arg Gly Trp Arg Met Arg Pro Glu Gln Leu Lys Gln Asp Asp Glu His
    130                 135                 140

Arg Ile Asn Asn Pro Glu Trp Tyr Gln Ser Glu Lys Leu Val Gly Gly
145                 150                 155                 160

Ala Leu Tyr Val Asp Leu Phe Ser Glu Asn Leu Gly Glu Leu Arg Lys
                165                 170                 175

Gln Ile Pro Tyr Phe Gln Asp Leu Gly Leu Ser Tyr Leu His Leu Met
            180                 185                 190

Pro Leu Phe Ala Val Arg Pro Gly Asn Asn Asp Gly Gly Tyr Ala Ile
        195                 200                 205

Ser Asn Tyr Arg Ser Val Asp Pro Arg Val Gly Thr Ile Asp Asp Leu
    210                 215                 220

Arg Leu Leu Ala Asp Asp Leu Arg Glu Ala Gly Ile Leu Leu Val Leu
225                 230                 235                 240

Asp Phe Val Phe Asn His Thr Ala Asp Asp His Tyr Trp Ala Gln Gln
                245                 250                 255

Ala Gln Ser Gly Asn Glu Glu Tyr Gln Lys Tyr Tyr Phe Ile Phe Pro
            260                 265                 270

Asp Arg Glu Val Pro Asp Gln Tyr Glu Arg Thr Leu Arg Glu Ile Phe
        275                 280                 285

Pro Thr Val Arg Arg Gly Asn Phe Thr Trp His Asp Gly Met Gln Gln
    290                 295                 300

Trp Val Trp Thr Thr Phe Asn Ser Phe Gln Trp Asp Leu Asn Tyr Arg
305                 310                 315                 320

Asn Pro Glu Val Phe Arg Ala Met Leu Ser Glu Met Leu Phe Ile Ala
                325                 330                 335

Asn Thr Gly Val Asp Leu Leu Arg Leu Asp Ala Val Ala Phe Ile Trp
            340                 345                 350

Lys Gln Met Gly Thr Asn Cys Glu Asn Leu Pro Glu Ala His Thr Leu
        355                 360                 365

Ile Arg Ala Phe Asn Ser Cys Val Arg Ile Ala Ala Pro Gly Leu Val
    370                 375                 380

Phe Lys Ser Glu Ala Ile Val His Pro Asp Asp Val Val Arg Tyr Ile
385                 390                 395                 400

Ser His Lys Glu Cys Gln Thr Ser Tyr Asn Pro Thr Leu Met Ala Leu
                405                 410                 415

Met Trp Glu Ser Leu Ala Thr Arg Asp Thr Lys Leu Leu Arg Arg Ser
            420                 425                 430

Leu Ala Arg Arg His Lys Leu Pro Glu Arg Thr Thr Trp Val Asn Tyr
        435                 440                 445

Leu Arg Cys His Asp Asp Ile Gly Trp Thr Phe Asp Asp Ala Asp Ala
    450                 455                 460

Ala Ala Val Gly Ile Asp Ala Phe Gly His Arg Gln Phe Leu Asn Ala
465                 470                 475                 480

Phe Tyr Thr Gly Gln Phe Glu Gly Ser Phe Ala Arg Gly Val Pro Phe
                485                 490                 495

Gln His Asn Pro Asp Asn Gly Asp Met Arg Ile Ser Gly Thr Leu Ala
            500                 505                 510

Ser Leu Ala Gly Leu Glu Gln Ala Ile Glu Ser Asp Arg Asp Asp Trp
        515                 520                 525

Lys Glu Leu Ala Ile Arg Arg Ile Leu Leu Leu Asn Ala Met Met Leu
    530                 535                 540

Ser Val Gly Gly Ile Pro Leu Val Tyr Leu Gly Glu Glu Trp Gly Ala
545                 550                 555                 560

Leu Asn Asp Tyr Asp Phe Val Lys Asp Pro Ala Lys Ala Gly Asp Thr
                565                 570                 575

Arg Trp Val His Arg Pro Lys Met Gln Trp Lys Phe Leu Glu Glu Leu
            580                 585                 590

Gly Asp Asp Gly Ser Ile Arg Gly Arg Ile Tyr Arg Ser Ile Arg Lys
        595                 600                 605

Leu Ile Ala Ile Arg Lys Met Thr Pro Ala Phe Gly Gly Leu Asp Met
    610                 615                 620

Glu Leu Leu Val Leu Glu Ser Pro His Leu Leu Gly Tyr Val Arg Gln
625                 630                 635                 640

His Glu Gly Asn Arg Val Ile Val Ile Ala Asn Phe Cys Glu His Ala
                645                 650                 655

Gln Glu Ile Asp Ser Asn Arg Leu Arg Thr Ala Gly Met Gly Arg Phe
            660                 665                 670

Phe Glu Asp Leu Tyr Ser Gly Gln Thr Ile Thr Thr Ser His Pro Leu
        675                 680                 685

Thr Leu Gly Pro Tyr Glu Phe Met Trp Leu His Arg Val
    690                 695                 700

<210>  12
<211>  650
<212>  PRT
<213>  Synechococcus sp.

<400>  12

Met Tyr Glu Gln Ile Ser His Ser Leu Leu Asn Ser Ile Leu Asp Glu
1               5                   10                  15

Leu Arg Pro Glu Ile Lys Arg Gln Asn Leu Arg Tyr Phe Tyr Thr Arg
            20                  25                  30

Leu Gly Ala Asn Phe Tyr Ala Ile His Ser Leu Phe Ser Thr Leu Tyr

35                  40                  45

Gly Asp Arg Glu Asp Phe Gln Pro Gln Met Val Arg Leu Val Glu Thr
    50                  55                  60

Met Ala Glu Gly Tyr Ile Tyr Arg Ser Glu Glu Ser Glu Arg Ile Asp
65                  70                  75                  80

Met Ser Arg Glu Gln Asp His Asn Trp Phe Leu Ser Gln Lys Trp Val
                85                  90                  95

Gly Met Ala Leu Tyr Thr Asn Gly Phe Ala Asn Asp Leu Gly Asp Leu
            100                 105                 110

Ala Gly Lys Ile Ser Tyr Phe Gln Glu Leu Gly Val Asn Met Val His
        115                 120                 125

Ile Met Pro Ile Leu Lys Cys Pro Ala Gly Gln Ser Asp Gly Gly Tyr
    130                 135                 140

Ala Ile Ser Asn Phe Arg Glu Ile Asp Ser Arg Val Gly Asp Leu Asn
145                 150                 155                 160

Asp Ile Gln Arg Ile Ser Glu Gln Phe Arg Lys Cys Gly Ile Leu Leu
                165                 170                 175

Val Leu Asp Ile Val Leu Asn His Thr Ser Asp Glu His Glu Trp Ala
            180                 185                 190

Leu Lys Ala Lys Asp Gly Asp Pro Arg Tyr Gln Asn Tyr Tyr Leu Thr
        195                 200                 205

Phe Glu Asp Arg Ser Val Pro Asp Val Phe Glu Gln Gly Met Pro Met
    210                 215                 220

Val Phe Pro Val Thr Asp Pro Gly Asn Phe Thr Trp Asn Glu Glu Met
225                 230                 235                 240

Lys Arg Trp Val Met Thr Val Phe His Asp Tyr Gln Trp Asp Leu Asn
                245                 250                 255

Tyr Ser Asn Pro Glu Val Phe Ile Glu Ile Leu Ser Ile Val Leu Phe
            260                 265                 270

Trp Ala Asn Gln Gly Val Asp Val Leu Arg Leu Asp Ala Val Ala Phe
        275                 280                 285

Leu Trp Lys Lys Leu Gly Ser Thr Cys Gln Asn Glu Arg Lys Ala His
    290                 295                 300

Leu Ile Leu Gln Leu Leu Lys Asp Cys Cys Gln Val Thr Ala Pro Gly

305                 310                 315                 320

Val Leu Phe Ile Ala Glu Ala Ile Val Ala Pro Ser Glu Val Ala Lys
                325                 330                 335

Tyr Phe Gly Glu Asp Ala Ile Val Ala Lys Glu Cys Glu Ile Ala Tyr
            340                 345                 350

Asn Ala Thr Leu Met Ala Leu Leu Trp Asp Gly Ile Ala Thr Arg Asn
        355                 360                 365

Thr Lys Leu Leu Arg Glu Gly Ile Lys Ser Leu Pro Thr Lys Leu Asp
    370                 375                 380

Arg Ala Thr Trp Leu Thr Tyr Val Arg Cys His Asp Asp Ile Gly Phe
385                 390                 395                 400

Gly Phe Asp Asp Lys Asp Ile Ala Ala Ala Gly Tyr Glu Pro His Ser
                405                 410                 415

His Arg Met Phe Leu Ile Asp Tyr Phe Thr Gly Lys Phe Ser Asp Thr
            420                 425                 430

Ser Arg Gly Leu Glu Phe Met Arg Asn Asp Ser Thr Gly Asp Ala Arg
        435                 440                 445

Ile Cys Gly Ser Leu Ala Ser Leu Ala Gly Leu Glu Ser Ala Leu Glu
    450                 455                 460

Lys Gly Asp Gln Gly Leu Ile Asp Met Ala Ile Ser Lys Ile Leu Leu
465                 470                 475                 480

Leu Asn Gly Val Ile Leu Ser Phe Gly Gly Ile Pro Leu Ile Tyr Asn
                485                 490                 495

Gly Asp Ala Leu Gly Gln Leu Asn Asp Tyr Ser Tyr Ile Asp Asp Pro
            500                 505                 510

Ser Lys Ser Asn Asp Ser Arg Trp Val His Arg Pro Lys Ile Asp Trp
        515                 520                 525

Glu Lys Ala Ala Leu Arg His Gln Arg Gly Thr Val Glu Cys Arg Ile
    530                 535                 540

Phe Thr Ala Met Lys Arg Met Ile Ser Ile Arg Lys Glu Ile Ser Ala
545                 550                 555                 560

Phe Ala Asp Phe Asn Asn Arg Glu Leu Met His Ser Asp Asn Gln His
                565                 570                 575

Leu Leu Cys Tyr Leu Arg Tyr Asn His Leu Arg Ser Ser Glu Lys Val

580                 585                 590

Phe Val Ile Ala Asn Phe Asp Asp His Pro Gln Gln Leu Glu Leu Glu
        595                 600                 605

Ser Tyr Ser Arg Tyr Gly Ile Asn Pro Arg Ala Asn Tyr Thr Asp Leu
    610                 615                 620

Tyr Ser Gly Ile Lys Pro Ser Gln Tyr Asp Gly Cys Ile Ala Leu Gln
625                 630                 635                 640

Gly Gly Gln Phe Tyr Trp Leu Ser Glu Ser
                645                 650

<210>  13
<211>  644
<212>  PRT
<213>  Xanthomonas axonopodis

<400>  13

Met Ser Thr Cys Pro Ile Asp Ser Pro Ala Leu Arg Ala Ala Phe Ala
1               5                   10                  15

Ala Pro Leu Asp Pro Gln His Ala Glu Val Leu Leu Ser Arg Tyr Asp
            20                  25                  30

Gln His Ala Ser Arg Leu Leu Asp Ala Leu His Ala Leu Tyr Gly Gln
        35                  40                  45

Arg Ala Asp Tyr Ala Ser Trp Leu Ala Gln Trp Leu Gly Glu Val Gly
    50                  55                  60

Asp Ile Ala Arg Gln Arg Pro Gln Ala Leu Gln Thr Leu Asp Ser Thr
65                  70                  75                  80

Arg His Ala Gly Trp Phe Gly Gln Gln His Met Leu Gly Tyr Ser Ala
                85                  90                  95

Tyr Ala Asp Arg Phe Ala Gly Thr Leu Gln Gly Val Ala Glu Arg Val
            100                 105                 110

Pro Tyr Leu Gln Glu Leu Gly Val Arg Tyr Leu His Leu Leu Pro Phe
        115                 120                 125

Leu Arg Ala Arg Ala Gly Asp Asn Asp Gly Gly Phe Ala Val Ser Asp
    130                 135                 140

Tyr Gly Gln Val Glu Pro Ser Leu Gly Ser Asn Asp Asp Leu Val Ala
145                 150                 155                 160

Leu Thr Ser Arg Leu Arg Glu Ala Gly Ile Ser Leu Cys Ala Asp Phe
                165                 170                 175

Val Leu Asn His Thr Ala Asp Asp His Ala Trp Ala Gln Ala Ala Arg
            180                 185                 190

Ala Gly Asp Ala Arg Tyr Leu Asp Tyr Tyr His His Phe Ala Asp Arg
        195                 200                 205

Thr Leu Pro Asp Arg Tyr Glu Ala Thr Leu Gly Gln Val Phe Pro His
    210                 215                 220

Thr Ala Pro Gly Asn Phe Thr Trp Val Asp Asp Thr Ala Gln Trp Met
225                 230                 235                 240

Trp Thr Thr Phe Tyr Pro Tyr Gln Trp Asp Leu Asn Trp Ser Asn Pro
                245                 250                 255

Ala Val Phe Gly Asp Met Ala Leu Ala Met Leu Arg Leu Ala Asn Leu
            260                 265                 270

Gly Val Glu Thr Phe Arg Leu Asp Ser Thr Ala Tyr Leu Trp Lys Arg
        275                 280                 285

Ile Gly Thr Asp Cys Met Asn Gln Pro Glu Ala His Thr Leu Leu Val
    290                 295                 300

Ala Leu Arg Ala Val Thr Asp Ile Val Ala Pro Ala Val Val Met Lys
305                 310                 315                 320

Ala Glu Ala Ile Val Pro Met Thr Gln Leu Pro Pro Tyr Phe Gly Ser
                325                 330                 335

Gly Val Asp Glu Gly His Glu Cys His Leu Ala Tyr His Ser Thr Leu
            340                 345                 350

Met Ala Ala Gly Trp Ser Ala Leu Ala Leu Gln Arg Gly Asp Ile Leu
        355                 360                 365

His Asn Val Ile Ala His Ser Pro Pro Leu Pro Arg His Cys Ala Trp
    370                 375                 380

Leu Ser Tyr Val Arg Cys His Asp Asp Ile Gly Trp Asn Val Leu Gln
385                 390                 395                 400

His Glu Ala Cys Gly Asn Ala Ala Gln Pro Pro Phe Ser Leu Arg Asp
                405                 410                 415

Val Ala Arg Phe Tyr Ala Asn Ala Val Pro Gly Ser Tyr Ala Arg Gly
            420                 425                 430

Glu Ser Phe Gln Ser Ser Gly Asp Gly Val His Gly Thr Asn Gly Met
        435                 440                 445

Ala Ala Ala Leu Ala Gly Ile Gln Ala Ala Gln Glu Ala Gly Asp Ala
    450                 455                 460

Ala Ala Leu Ala Val Ala Val Asp Arg Leu Val Leu Leu Tyr Ala Ile
465                 470                 475                 480

Ala Leu Ala Met Pro Gly Val Pro Leu Ile Tyr Met Gly Asp Glu Leu
                485                 490                 495

Ala Met Val Asn Asp Pro Gly Tyr Arg His Asp Pro His Arg Gln His
            500                 505                 510

Glu Gly Arg Trp Leu His Arg Pro Ala Met Asp Trp Gln Leu Ala Ala
        515                 520                 525

Gln Arg His Asp Ala Asn Ser Leu Ser Gly Thr Val Tyr Arg Arg Leu
    530                 535                 540

Arg Gly Leu Ile Arg Gln Arg Ala Ala Leu Gly Ala Leu Ala Ala Asp
545                 550                 555                 560

Gln Ala Leu Ala Ser Ile Ala Leu Asn Asp Pro Arg Val Phe Val Leu
                565                 570                 575

Thr Arg Gly Asp Ser Phe Ile Ala Leu His Asn Phe Ser Asp Gln Pro
            580                 585                 590

Leu Asp Val Glu Leu Ala Ala Ile Gly Ile Asp Gly Trp Thr Leu Leu
        595                 600                 605

Ala Ile Asp Asp Ala Ile Gly Gly Ala Ala Ala Arg Gly Asp Gly Ser
    610                 615                 620

Ile Val Leu Pro Pro Tyr Gly Val Arg Trp Leu Gln Arg Gly Thr Glu
625                 630                 635                 640

His Ala Pro Glu

<210>  14
<211>  637
<212>  PRT
<213>  Xanthomonas campestris

<400>  14

Met Ile Ala Ser Ser Pro Ile Asp Ala Ala Ala Leu Arg Ala Ser Val
1               5                   10                  15

Ala Ala Ala Leu Asp Pro Ser Thr Ala Val Ala Thr Leu Ala Arg Phe
            20                  25                  30

Asp Thr His Ala Pro Arg Leu Leu Asp Ala Leu Ser Thr Leu Tyr Gly
        35                  40                  45

Asp His Ala Asp Tyr Val Thr Trp Leu Pro Gln Trp Leu Ser Ala Leu
    50                  55                  60

Gly Val Val Ala Gln Ala Arg Pro Ala Ala Leu Arg His Leu Asp Asp
65                  70                  75                  80

Ser Arg Ala Pro Gly Trp Phe Gly Gln Gln Asp Met Leu Gly Tyr Ser
                85                  90                  95

Ala Tyr Val Asp Arg Phe Ala Gly Thr Leu Arg Gly Val Ala Glu Arg
            100                 105                 110

Val Pro Tyr Leu Gln Glu Leu Gly Val Arg Tyr Leu His Leu Leu Pro
        115                 120                 125

Phe Leu Arg Ala Arg Ala Gly Asp Asn Asp Gly Gly Phe Ala Val Ser
    130                 135                 140

Asp Tyr Gly Gln Val Glu Pro Ala Leu Gly Ser Asn Asp Asp Leu Val
145                 150                 155                 160

Ala Leu Thr Ala Arg Leu Arg Ala Ala Asn Ile Ser Leu Cys Ala Asp
                165                 170                 175

Phe Val Leu Asn His Thr Ala Asp Asp His Ala Trp Ala Gln Ala Ala
            180                 185                 190

Arg Ala Gly Asp Thr Arg Tyr Leu Asp Tyr Tyr His His Phe Ala Asp
        195                 200                 205

Arg Asn Ala Pro Asp Gln Tyr Asp Thr Thr Leu Val Gln Val Phe Pro
    210                 215                 220

Gln Thr Ala Pro Gly Asn Phe Thr Trp Val Asp Glu Thr Arg Gln Trp
225                 230                 235                 240

Met Trp Thr Thr Phe Tyr Pro Tyr Gln Trp Asp Leu Asn Trp Ser Asn
                245                 250                 255

Pro Ala Val Phe Gly Glu Met Ala Leu Ala Met Leu Glu Leu Ala Asn
            260                 265                 270

Leu Gly Val Glu Ala Phe Arg Leu Asp Ser Thr Ala Tyr Leu Trp Lys
        275                 280                 285

Arg Pro Gly Thr Asn Cys Met Asn Gln Pro Glu Ala His Thr Ile Leu
    290                 295                 300

Val Ala Leu Arg Ala Val Ala Asp Ile Val Ala Pro Ser Val Val Met
305                 310                 315                 320

Lys Ala Glu Ala Ile Val Pro Met Ala Glu Leu Pro Pro Tyr Phe Gly
                325                 330                 335

Ser Gly Val Gln Arg Gly His Glu Cys His Leu Ala Tyr His Ser Thr
            340                 345                 350

Leu Met Ala Ala Gly Trp Ser Ala Leu Ala Leu Gln Arg Gly Asp Ile
        355                 360                 365

Leu Gln Asp Val Ile Ala His Ser Pro Pro Leu Pro Pro Asn Cys Ala
    370                 375                 380

Trp Leu Ser Tyr Val Arg Cys His Asp Asp Ile Gly Trp Asn Val Leu
385                 390                 395                 400

Gln His Glu Ala Ala Gly Thr Ala Ala Gln Pro Pro Phe Ser Leu Arg
                405                 410                 415

Glu Val Ala Gln Phe Tyr Ala Asn Ala Val Pro Gly Ser Tyr Ala Arg
            420                 425                 430

Gly Glu Ser Phe Gln Ser Ser Gly Asp Gly Val His Gly Thr Asn Gly
        435                 440                 445

Met Ser Ala Ala Leu Val Gly Val Gln Ala Ala His Glu His Ala Asp
    450                 455                 460

Ala Ala Ala Ala Ala Arg Ala Val Asp Arg Leu Val Leu Leu Tyr Ala
465                 470                 475                 480

Val Ser Leu Ala Met Pro Gly Val Pro Leu Ile Tyr Met Gly Asp Glu
                485                 490                 495

Leu Ala Leu Pro Asn Asp Thr Ala Tyr Leu Asp Asp Ala Gln Arg Arg
            500                 505                 510

His Glu Gly Arg Trp Leu His Arg Pro Ala Met Ala Trp Glu Leu Ala
        515                 520                 525

Ala Gln Arg His Asp Ala Ser Thr Leu Ala Gly Thr Val Tyr Thr Arg
    530                 535                 540

Leu Arg Ala Leu Ile Arg Leu Arg Ala Gly Leu Pro Ala Leu Ala Ala
545                 550                 555                 560

Thr Gln Ser Leu Gly Ser Val Ala Leu Gly Asp Ala Arg Leu Phe Ala
                565                 570                 575

Leu Thr Arg Gly Asp Ser Phe Leu Ala Val His Asn Phe Ser Asp Val
            580                 585                 590

Pro Leu Pro Val Asp Leu Thr Gln Thr Gly His Ala Leu Trp Ala Val
        595                 600                 605

Leu Asp Thr Asp Gly Thr Gly Asp Ala Pro Glu Pro His Thr Glu Leu
    610                 615                 620

Leu Leu Pro Ala Tyr Gly Val Arg Trp Leu Gln Arg Arg
625                 630                 635

<210>  15
<211>  584
<212>  PRT
<213>  Caulobacter crescentus

<400>  15

Met Glu Ser Arg Phe Ala Lys Leu Tyr Gly Ala Asp Ala Arg Gly Pro
1               5                   10                  15

Ala Val Leu Glu Arg Leu Lys Thr Asn Leu Leu Lys Ala Ala His Ala
            20                  25                  30

Arg Pro Glu Pro Leu Arg Ala Leu Asp Ala Ala Arg Ala Ala Asp Pro
        35                  40                  45

Ala Trp Leu His Ala Pro Gly Gln Thr Ala Tyr Thr Phe Tyr Val Asp
    50                  55                  60

Arg Phe Ala Gly Asp Leu Asn Gly Val Arg Gly Lys Leu Asp Tyr Leu
65                  70                  75                  80

Thr Glu Leu Gly Val Arg Trp Leu His Pro Leu Pro Leu Leu Glu Pro
                85                  90                  95

Arg Pro Gly Asp Ser Asp Gly Gly Phe Ala Val Ala Asp Tyr Arg Lys
            100                 105                 110

Val Asp Pro Arg Leu Gly Thr Ile Asp Asp Leu Glu Ala Leu Ala Gly
        115                 120                 125

Asp Leu Arg Gln Arg Asp Met Gly Leu Ile Leu Asp Val Val Cys Asn
    130                 135                 140

His Thr Ala Arg Glu His Ala Trp Ala Ala Lys Ala Arg Ala Gly Asp
145                 150                 155                 160

Pro Ala Tyr Arg Asp Tyr Tyr Ile Val Leu Pro Asp Ala Gln Ser Ala
                165                 170                 175

Ala Ala Arg Asp Arg Glu Leu Ile Asp Val Phe Pro Asp Thr Ala Pro
            180                 185                 190

Gly Ser Phe Thr Tyr Asp Ala Ala Met Gly Gly Tyr Val Trp Thr Thr
        195                 200                 205

Phe Tyr Pro Phe Gln Trp Asp Leu Asn Tyr Ala Asn Pro Ala Val Phe
    210                 215                 220

Ala Glu Met Leu Glu Val Leu Ile Phe Leu Ala Ala Lys Gly Ala Gln
225                 230                 235                 240

Gly Phe Arg Leu Asp Ser Ala Pro Phe Leu Trp Lys Gln Ala Gly Thr
                245                 250                 255

Thr Cys Arg Asn Leu Pro Gln Thr Tyr Glu Ile Val Glu Ala Trp Arg
            260                 265                 270

Ala Ala Leu Ser Ile Val Ala Pro Gly Val Val Leu Leu Ala Glu Ala
        275                 280                 285

Ile Glu Ser Val Glu Asp Val Leu Pro Phe Phe Gly Gly Glu Glu Ser
    290                 295                 300

Gly Cys Asn Leu Ala Tyr Asn Asn Val Val Met Thr Ala Leu Trp Ala
305                 310                 315                 320

Ala Leu Ala Asp Gly Asp Ala Val Ile Ala Arg Arg Cys Leu Ala Val
                325                 330                 335

Ala Ala Arg Lys Pro Ala Gln Gly Ala Trp Leu Asn Tyr Val Arg Cys
            340                 345                 350

His Asp Asp Leu Ile Trp Asn Ala Leu Ala Ala Tyr Ala Pro Ala Ser
        355                 360                 365

Asp Leu Arg Arg Trp Ser Asn Ala Tyr Gly Asn Gly Glu Gly Phe Ser
    370                 375                 380

Arg Gly Arg Ala Phe Gln Thr Ala Glu Gly Gly Val Pro Ser Thr Asn
385                 390                 395                 400

Gly Met Ala Ala Ala Leu Ala Gly Leu Thr Ala Asp Ala Asp Gly Asp
                405                 410                 415

Cys Leu Gly Ala Arg Arg Leu Arg Leu Leu Tyr Gly Ile Ile His Ala
            420                 425                 430

Leu Asp Gly Trp Pro Leu Ile Tyr Met Gly Asp Glu Ile Gly Leu Asp
        435                 440                 445

Asn Asp Glu Ala Tyr Gln Asp Asp Pro Leu Arg Ala Gly Asp Gly Arg
    450                 455                 460

Trp Leu His Arg Pro Gln Met Asp Trp Ser Leu Ala Glu Arg Arg Gly
465                 470                 475                 480

Glu Ala Gly Ala Leu Gln Ala Asp Leu Phe Ala Thr Phe Ala Arg Leu
                485                 490                 495

Gly Gln Arg Ala Arg Arg Leu Ala Thr Leu Gly Val Ala Gly Pro Ala
            500                 505                 510

Arg Pro Val Glu Val Ser Ser Pro Ala Val Leu Ala Phe Leu Arg Asp
        515                 520                 525

Glu Gly Ala Arg Pro Phe Leu Cys Val Ala Asn Val Ser Asp Ala Pro
    530                 535                 540

Gln Asp Phe Glu Leu Pro Pro Ala Phe Ala Gln Gly Ala Glu Asp Val
545                 550                 555                 560

Leu Asp Gly Ala Pro Ser Pro Ala Gly Ala Val Ser Leu Pro Pro Tyr
                565                 570                 575

Gly Ile Thr Trp Leu Val Ala Arg
            580

## Description

The invention relates to the field of the production and application of starch. The invention particularly relates to methods and means for producing starch having at least one altered characteristic, comprising introducing in a starch producing plant an amylosucrase encoding sequence.

Starch is the most abundant storage reserve carbohydrate in plants. Starch is deposited as crystalline granules, which generally consist of two polysaccharides, amylose and amylopectin. In plant storage organs, starch biosynthesis takes place within the amyloplast. Sucrose is the starting point of starch biosynthesis. Many different reactions and enzymes are further known to be involved in the starch biosynthesis. Although in recent years starch structure has been studied intensively, some mechanisms in starch biosynthesis have remained elusive. One of the mechanisms that has not been sufficiently clarified is the mechanism that establishes the size of starch granules.

Starch is an important raw material for diverse foods and industrial applications. A few examples of industrial applications are the use as a thickening agent in food, use for surface sizing in paper manufacturing and use for creating a slow-release matrix in which therapeutic compounds are dispersed. The suitability of starch for specific applications is determined by its characteristics such as for example its granule size, physico-chemical properties and the presence of non-starch components, such as protein and lipid. Characteristics of starch for instance influence the selection and processing of starch and are thus of importance to starch processing companies. Therefore, the industry has considerable interest in starches with diverse characteristics. As the starch industry has an interest in starches with diverse characteristics there is a demand for methods and means for producing starch with an altered characteristic.

The invention now provides methods and means for producing starch having at least one altered characteristic. The invention provided herein is in one aspect based upon the finding that introduction of an amylosucrase encoding sequence in an amyloplast of a starch producing plant leads to at least one altered characteristic of starch produced by said plant. The invention in one embodiment provides a method for modifying a starch producing plant, comprising introducing a nucleic acid comprising an amylosucrase encoding sequence functionally linked to a granule-targeting sequence and functionally linked to a promoter sequence active in a starch storage organ of said plant into suitable plant material of said plant. In another embodiment the invention provides a method for producing starch having at least one altered characteristic, comprising introducing a nucleic acid comprising an amylosucrase encoding sequence functionally linked to a granule-targeting sequence and functionally linked to a promoter sequence active in a starch storage organ of a plant into suitable plant material of said plant and producing a plant from said material, further comprising allowing said plant to grow and harvesting starch from said plant. Starch should be understood to have an altered characteristic when the characteristic of starch is altered compared to starch from a plant of the same species wherein an amylosucrase encoding sequence has not been introduced. A characteristic is defined as any parameter of starch, said characteristic thus for instance relates to structure and/or physical properties and/or chemical properties. A plant and/or starch producing plant is for the purpose of the invention defined as any plant wherein starch is produced, preferably said starch is produced in a specialized plastid, i.e. the amyloplast. Non-limiting examples of starch producing plants are root crops such as cassava, potato, sweet potato, taro, yam, beet or turnip; cereals such as maize, wheat, rice, sorghum or barley; fruit producing species such as banana, apple, tomato or pear; meal crops such as soybean or pea; oilseed crops such as rapeseed, canola, sunflower, oil palm, coconut, linseed or groundnut. A preferred starch producing plant is a potato plant, more preferably an amylose-containing potato plant such as for example cultivar Kardal. Suitable plant material is for the purpose of the invention defined as any plant material wherein an amylosucrase encoding sequence and further sequences can be introduced. Suitable plant material is either naturally occurring material or artificially synthesized material. Naturally occurring plant material is for instance material of a very early stadium of a plant, such as material of a seed or a small shoot. A preferred suitable plant material is a protoplast. A protoplast is for the purpose of the invention defined as a plant cell from which the cell wall is completely or partially removed using either mechanical or chemical, such as enzymatic, means. A protoplast can for instance be regenerated into a whole plant using micropropagation. Allowing a plant to grow should be understood as keeping and/or placing said plant in circumstances wherein it is capable of growing.

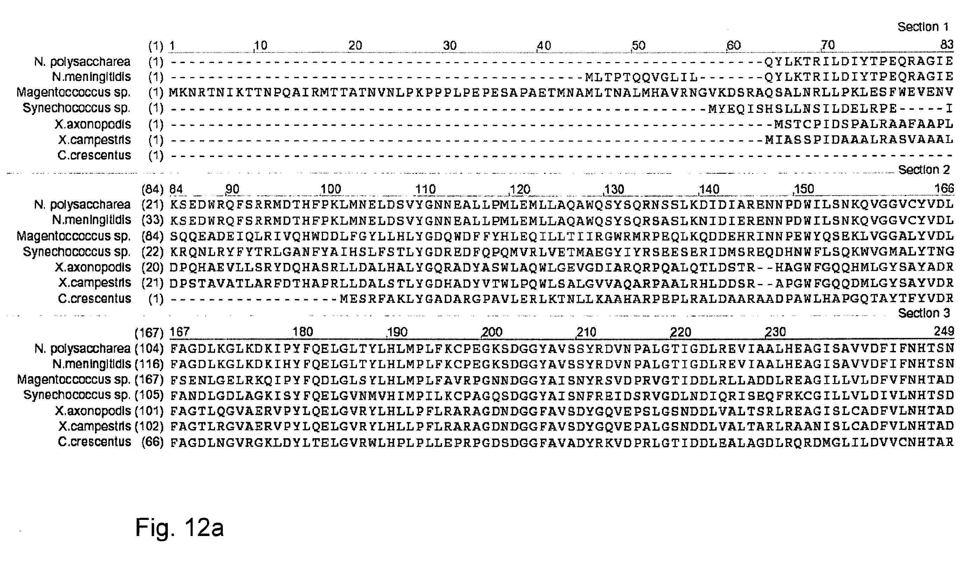

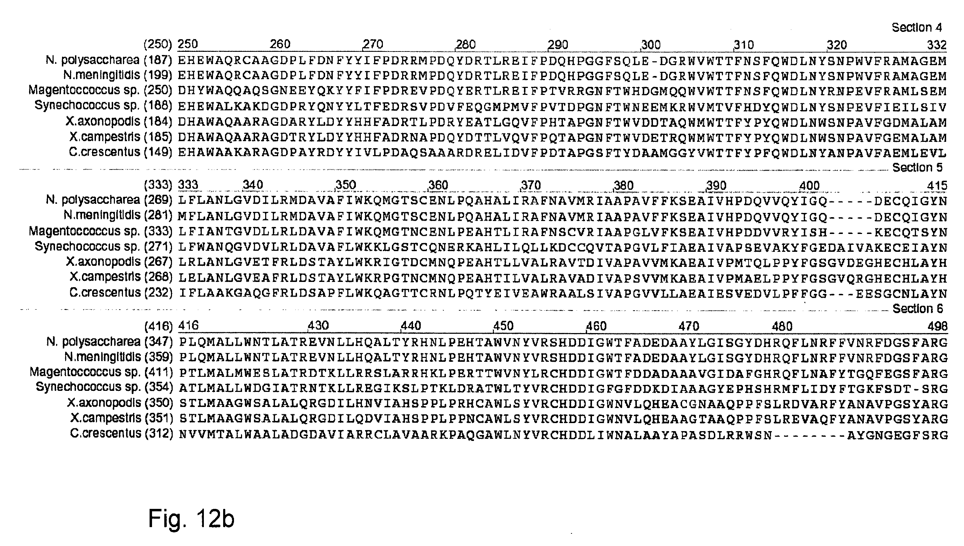

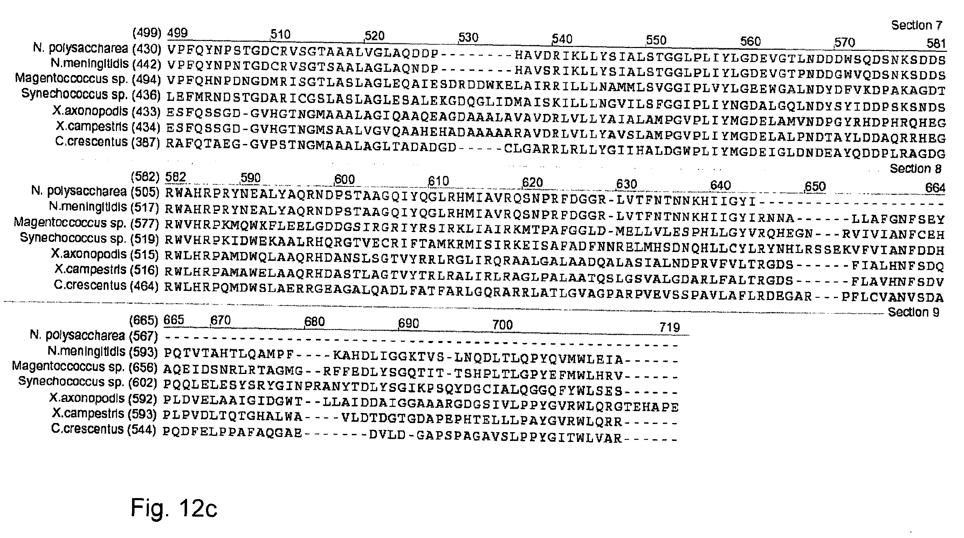

Amylosucrase is for the purpose of the invention intended to include any amylosucrase produced by an organism, preferably a bacterium, and/or any natural, synthetic or recombinant compound that has an amylosucrase activity. A compound that has amylosucrase activity is for instance an amylosucrase that has been truncated. Such a truncated amylosucrase is for instance produced by providing an organism with a truncated gene that encodes said amylosucrase. Said truncated gene encodes at least a functional amylosucrase and optionally further sequences. Amylosucrase is a member of the glycoside hydrolase GH13 family. More specifically, it is a hexosyltransferase that is for instance produced by different bacteria, for example non-pathogenic bacteria from the species Neisseria, Magentococcus species and Synechococcus species. An amylosucrase encoding sequence according to the invention preferably encodes an amylosucrase that is preferably at least 70%, more preferably at least 80%, more preferably at least 90%, more preferably at least 95%, most preferably more than 95% homologous or identical to an amylosucrase as produced by a Neisseria bacterium, wherein said Neisseria is preferably Neisseria polysaccharea (De Montalk et al. 1999). Preferably an amylosucrase encoding sequence according to the invention encodes an amylosucrase that is at least 70%, more preferably at least 80%, more preferably at least 90%, more preferably at least 95%, most preferably more than 95% homologous or identical to the amylosucrase sequence as shown in figure 12. In one embodiment of the invention, one or more nucleic acids in said amylosucrase encoding sequence are replaced by conservative substitutions.

An amylosucrase encoding sequence is typically a nucleic acid sequence. A nucleic acid for the purpose of the invention includes typically, though not necessarily, DNA or RNA. A nucleic acid preferably comprises nucleotides A, C, G, T and/or U. These nucleotides are either ribonucleotides, deoxyribonucleotides and/or other nucleotide analogues, such as synthetic nucleotide analogues. A nucleotide analogue is for the purpose of the invention amongst others intended to include a peptide nucleic acid (PNA), a locked nucleic acid (LNA), or alternatively a backbone- or sugar-modified ribonucleotide or deoxyribonucleotide. Furthermore, the nucleotides in a nucleic acid sequence of the invention are optionally substituted by corresponding nucleotides that are capable of forming analogous H-bonds to a complementary nucleic acid sequence. An example of such a substitution is the substitution of U by T.

Introducing an amylosucrase encoding sequence in a starch producing plant is defined as providing an amylosucrase encoding sequence to said plant such that said sequence is internalised in a cell of said plant. A condition wherein said plant initially, before introduction of an amylosucrase encoding sequence, did not comprise an amylosucrase encoding sequence is covered herein as well as a condition wherein said plant initially did comprise an amylosucrase encoding sequence. Introduction of an amylosucrase encoding sequence functionally linked to other sequences such as a granule-targeting sequence and a promoter sequence, may be achieved by any means for introducing a nucleic acid in a cell available to a person skilled in the art. Means for introducing a nucleic acid in a cell include gene transfer and transformation methods. Examples of gene transfer and transformation methods are protoplast transformation through calcium-, polyethylene glycol (PEG) or electroporation-mediated uptake of DNA, electroporation of plant tissues, microinjection, silicon carbide mediated DNA uptake and microprojectile bombardment. A preferred transformation method is a vector mediated transformation and/or an *Agrobacterium*-mediated transformation method. A very preferred transformation method is an *Agrobacterium tumefaciens* mediated transformation method. An introduced sequence is preferably stably expressed in said plant. Therefore, a method of the invention preferably comprises integration of said sequence into the genome of a cell of said plant. Integration ensures a long-term, reproducible as well as a defined gene expression.

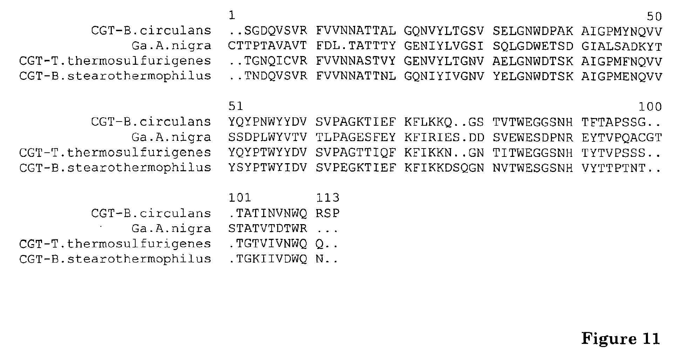

An amylosucrase encoding sequence according to the invention is preferably functionally linked to a granule-targeting sequence. A granule-targeting sequence is any sequence that targets the starch granule, i.e. selectively aims for and/or binds to a starch granule. Preferred examples of said granule-targeting sequences comprise potato granule-bound starch synthase I (GBSS I) and a starch-binding domain (SBD). Further examples of granule targeting sequences that can be used are those parts of enzymes that are linked to starch under biological conditions and/or binding domains from enzymes that are known to degrade starch. In a preferred embodiment of the invention said granule-targeting sequence comprises an SBD encoding sequence. An SBD belongs to the family of carbohydrate-binding modules (CBMs). A CBM is usually defined as contiguous amino acid sequence within a carbohydrate-active enzyme with a discreet fold having carbohydrate-binding activity. A few exceptions to this definition are CBMs in cellulosomal scaffolding proteins and independent putative CBMs. The requirement of CBMs existing as modules within larger enzymes sets this class of carbohydrate-binding protein apart from other non-catalytic sugar binding proteins such as lectins and sugar transport proteins. An SBD for the purpose of the invention is preferably a member of the alpha glucan binding domain family, more preferably a member of the alpha glucan binding domain family 20, 21, 25, 26, 34 or 41. An SBD encoding sequence is typically a nucleic acid sequence. SBD's are in nature primarily involved in a context of degradation of starch as the SBD's are in nature generally bound to degrading enzymes. A method of the invention however, uses SBD's in a different context, namely in starch biosynthesis. SBD's generally have a length of approximately 100 amino acids. Although the amino acid sequences of SBD's are not very well conserved among different enzymes as well as among different species because of their folding and putative 3D structure they are classified as similar (Machovic, 2005). SBD's for the purpose of the invention are for instance derived from α-amylase, β-amylase, glucoamylase or CGTase. Species producing an SBD comprising enzyme are for instance Aspergillus niger, Bacillus circulans, Streptomyces limosus, Clostridium thermosulfurogenes, Pseudomomnas stutzeri or Klebsiella pneumoniae. An SBD encoding sequence according to the invention encodes an SBD that is preferably at least 70%, more preferably at least 80%, more preferably at least 90%, more preferably at least 95%, most preferably more than 95% homologous or identical to an SBD as produced by a Bacillus bacterium, wherein said Bacillus bacterium is preferably a Bacillus circulans bacterium and wherein said SBD encoding sequence is preferably of cyclodextrin glycosyltransferase (Lawson et al.1994). Preferably an SBD encoding sequence according to the invention encodes an SBD that is at least 70%, more preferably at least 80%, more preferably at least 90%, more preferably at least 95%, most preferably more than 95% homologous or identical to the SBD shown in figure 11. In one embodiment of the invention, one or more nucleic acids in said SBD encoding sequence are replaced by conservative substitution. In a preferred embodiment an SBD is an SBD of cyclodextrin glycosyltransferase and/or an SBD from Bacillus circulans. In another preferred embodiment an SBD or other granule-targeting sequence according to the invention is approximately or exactly the same size as an SBD of cyclodextrin glycosyltransferase from Bacillus circulans, i.e. 10-14 kDa, preferably 12 kDa. In one embodiment of the invention a nucleic acid comprises more than one granule targeting sequence, for instance two, three, four or five.

A promoter sequence active in a starch storage organ of said plant according to the invention is defined herein as a sequence, preferably a nucleic acid sequence, that enables a gene to be transcribed, wherein said transcription will at least occur in and/or adjacent to a said starch storage organ. Said promoter is in one embodiment of the invention specific for said starch storage organ. Said specificity of said promoter is either for said starch storage alone or is specific for said starch storage organ and other elements of said plant. Alternatively said promoter is a generic promoter. In one embodiment of the invention said promoter is an inducible promoter. A starch storage organ is for the purpose of the invention defined as a part of a plant wherein starch is accumulated. Examples of such parts are a tuber, stem, root, seed or seed endosperm. A preferred part is a tuber, a preferred tuber is a potato. Examples of tissue specific promoters are listed in table 2, a preferred promoter is a potato patatin promoter.

The invention in a preferred embodiment provides a method according to the invention, wherein said nucleic acid is further functionally linked to an amyloplast-targeting sequence. In a further preferred embodiment of the invention a method according to the invention is provided wherein said amyloplast-targeting sequence comprises a transit peptide encoding sequence. An amyloplast-targeting sequence comprising a transit peptide encoding sequence is for the purpose of the invention defined as a sequence that is at least specific for an amyloplast and wherein said transit peptide encoding sequence directs said sequence specifically to an amyloplast. A preferred example of an amyloplast-targeting sequence is a ferredoxin transit peptide encoding sequence. Further examples of a transit peptide are a potato GBSSI transit peptide, a transitpeptide of the branching enzymes (BE I and/or BE II), an R-Enzyme (a water dikinase) and a soluble starch synthase I, II and/or III, and/or different isoforms (Stisa1, Stisa2 and Stisa3) of isoamylases.

Functionally linked sequences are for the purpose of the invention intended to include sequences that are linked such that all involved sequences are simultaneously directed to the same location on introduction in a starch producing plant and such that all involved sequences are capable of exerting their specific function(s) in coordination with each other. Said functional linkage may be achieved by different types of binding. Further, said sequences may be directly linked to each other or are alternatively linked indirectly. An example of directly linked sequences are sequences that are adjacently linked to each other in one nucleic acid construct. Alternatively, sequences are indirectly linked such that, for instance, the sequences are in the same nucleic acid construct but one or more other sequences are in between the functionally linked sequences or such that the sequences are in different nucleic acid constructs and functionally linked.

In one embodiment of a method of the invention additional sequences are introduced in a starch producing plant. Said additional sequences are either functionally linked to previously mentioned nucleic acid sequences or not functionally linked but introduced simultaneously and/or before and/or after introduction of said previously mentioned sequences. Said additional sequences for instance encode compounds that establish further modifications in said starch and/or said plant. Said compounds are for instance enzymes such as starch branching enzyme, acetyltransferase, methylating enzyme and R-enzyme. Said R-enzyme is a glucan water dikinase, GWD, involved in the phosphorylation of starch. In one embodiment of the invention said compound is a protein, as a starch storage organ of a cell can advantageously be used for protein production. Other additional sequences are sequences that are necessary and/or aid in the expression of introduced sequences, said additional sequences are preferably functionally linked to said introduced sequences. Examples of such additional sequences are a promoter and a terminator sequence.

A method of the invention preferably comprises a selection step. Said selection step is performed after introduction of sequences according to the invention and comprises selection of a plant wherein said introduced sequences are expressed, preferably stably expressed. A variety of systems for selecting a plant and/or a cell wherein a sequence is introduced and/or expressed are available to a person skilled in the art. In order to facilitate said selection, a nucleic acid is in a method of the invention preferably functionally linked to a selection marker. Alternatively, a selection marker is not functionally linked to said nucleic acid but is introduced simultaneously with said nucleic acid, such as for instance co-expressed on a second, co-transfected vector. A selection marker comprises a sequence that aids in determining whether a sequence has been introduced and/or is stably expressed in a plant and/or cell. A selection marker preferably comprises a resistance conferring sequence, such as a sequence conferring resistance to antibiotics. Examples of such resistance conferring sequences are sequences encoding neomycin phosphotransferase, dihydrofolate reductase (DHFR), or glutamine synthetase. Said resistance conferring sequences are in one embodiment introduced as a selection cassette, i.e. introduced comprising at least a promoter sequence specific for inducing expression of said resistance conferring sequence and further comprising at least a terminator sequence for ending transcription of said introduced resistance conferring sequence. In a preferred embodiment of a method of the invention a vector is used for introducing sequences in a starch producing plant. Said antibiotics resistance conferring sequence is in this embodiment preferably comprised in said vector. Said vector further preferably comprises other sequences, wherein said other sequences are preferably for use in cloning with bacteriophages, such as polylinker sequences. A preferred example of a vector is pBlueScript, which is a commercially available vector. In another embodiment of the invention, a method of the invention is performed without the use of a selection marker. In this embodiment preferably a marker free transformation method is used such as for instance described in WO 03/010319.

In a preferred embodiment the invention provides a method according to the invention, wherein said nucleic acid comprising an amylosucrase encoding sequence functionally linked to a granule-targeting sequence and functionally linked to a promoter sequence encodes a fusion protein. A fusion protein is for the purpose of the invention defined as a protein created through genetic engineering from two or more proteins/peptides. This is achieved by creating a fusion sequence. In order to create a fusion sequence the stop codon from the nucleic acid sequence of the first protein is preferably removed and subsequently the nucleic acid sequence of the second protein is appended in frame. When two sequences are protein and/or peptide encoding sequences, linker or spacer peptide encoding sequences are preferably added in order to promote independent folding of the protein and/or peptide parts as encoded by said sequences in the resulting fusion protein. The invention thus in one embodiment provides a nucleic acid comprising an amylosucrase encoding sequence, a starch binding domain (SBD) encoding sequence and a promoter sequence. In one embodiment of the invention said promoter comprised in said nucleic acid is a tuber specific promoter. In a further embodiment the invention provides a fusion protein comprising amylosucrase and a starch binding domain (SBD). Said starch binding domain of said nucleic acid or said fusion protein is preferably at least 70%, more preferably at least 80%, more preferably at least 90%, more preferably at least 95%, most preferably more than 95% homologous or identical to the SBD sequence shown in figure 11. The invention further provides a fusion protein according to the invention, further comprising an amyloplast transit peptide. In a preferred embodiment the invention provides a fusion protein according to the invention, wherein said starch binding domain comprises a starch binding domain of a cyclodextrin glycosyltransferase.

The invention in a preferred embodiment provides a fusion protein wherein a sequence encoded by a granule targeting sequence is located on the N- or C-terminal side of an amylosucrase. Said granule targeting sequence is preferably a starch binding domain. In a preferred embodiment of the invention said granule targeting sequence is located on the N-terminal side of an amylosucrase. Therefore, in a preferred embodiment of the invention, a method according to the invention comprises introducing in a starch producing plant a nucleic acid comprising an amylosucrase encoding sequence functionally linked to a granule-targeting sequence and functionally linked to a promoter sequence active in a starch storage organ of said plant, wherein said functionally linked granule-targeting sequence is introduced closer to the promoter than said amylosucrase encoding sequence. In an example of one embodiment of the invention it is illustrated that the location of the granule targeting sequence, which is an SBD in this example, has a significant effect on characteristics of the resulting fusion protein. Said location in this embodiment of the invention for instance influences the accumulation level of fusion proteins in a plant. Said accumulation level is in this embodiment further correlated to the percentage of altered starch granules. In the present embodiment of the invention a higher level of accumulation results in a higher percentage of altered starch granules.

An important characteristic that is altered by methods and means provided by the invention is starch granule size. Granule size is an important factor for many applications of starch. Depending on the biological source, starch granule size may vary from less than 1 µm to more than 100 µm. In potato for instance, starch granule size ranges from 5 to 100 µm. Since granule size is such an important factor in many industrial applications, there is an industrial interest in altering starch granule size. It is possible to obtain starch granules with a specific desired size by separation techniques, such as wind sifting, dry or wet sieving or separation by hydrocyclones, as a part of the starch processing. However, such a separation requires an additional step in processing, which results in a loss of efficiency. Therefore, it would be advantageous if starch granule size could be controlled by modifying the production of starch in a plant such that the size of a starch granule produced by the plant is altered. The invention provides such a controlling method by providing a method for producing starch having at least one altered characteristic, comprising introducing in a starch producing plant an amylosucrase encoding sequence. The biological function of amylosucrase is the synthesis of amylose-like polymers from sucrose. A person skilled in the art would expect that introduction of an amylosucrase encoding sequence in a starch producing plant would have an effect on amylose content of a starch granule. The invention however discloses experiments wherein no significant changes in amylose content resulted after introduction of an amylosucrase encoding sequence in a starch producing plant. Granule size on the other hand did change significantly. Therefore, the invention in one embodiment provides a method for producing starch having at least one altered characteristic, comprising introducing a nucleic acid comprising an amylosucrase encoding sequence functionally linked to a granule-targeting sequence and functionally linked to a promoter sequence active in a starch storage organ of a plant into suitable plant material of said plant and producing a plant from said material, further comprising allowing said plant to grow and harvesting starch from said plant, wherein said alteration comprises an increase in starch granule size. In another embodiment the invention provides a method for modifying a starch producing plant, comprising introducing a nucleic acid comprising an amylosucrase encoding sequence functionally linked to a granule-targeting sequence and functionally linked to a promoter sequence active in a starch storage organ of said plant into suitable plant material of said plant, wherein said modification comprises an increase in starch granule size. Said increase in starch granule size is defined as an increase relative to a starch granule size that a plant of the same and/or comparable species produces without introduction of an amylosucrase encoding sequence, i.e. control plant. Said increase may be an absolute increase, i.e. said control plant does not or generally does not produce starch granules of starch having said increased granule size and/or said increase may be an increase in mean granule size. A method of the invention preferably increases a starch granule size by 1.5, more preferably by 1.6, more preferably by 1.7, more preferably by 1.8, more preferably by 1.9, most preferably by 2.0 or more. An example of the invention illustrates the increase in granule size by expression of amylosucrase fusion proteins. Expression of a fusion protein comprising an amylosucrase and a starch binding domain in a potato plant induced an approximately two fold increase in starch granule size. An increased granule size is for example often desirable for maize and cassava starch because an increased starch granule size for instance improves wet-milling efficiency and thus starch yield.

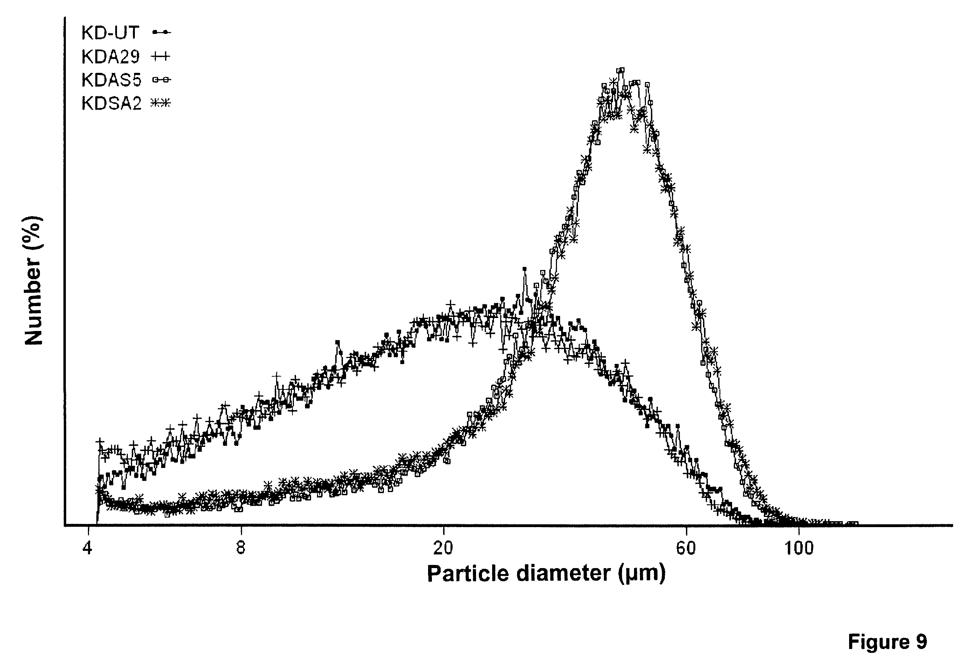

As an alternative for absolute increase, said increase is a relative increase such as an altered granule size distribution, wherein said distribution is shifted towards larger granules. Therefore, the invention in one embodiment provides a method according to the invention, wherein said modification and/or alteration comprises a shift in a distribution of starch granule size produced by said plant towards larger granules. A starch producing plant produces starch granules of different sizes. Each starch type produced by a plant has its characteristic distribution of sizes. Therefore, starch granule size is advantageously expressed as a distribution of sizes. Said distribution of sizes for instance demonstrates a uniformity of starch granule size. A method of the invention in one embodiment increases said uniformity of starch granule size, as for instance illustrated by figure 9 of the examples. Uniformity of starch granule size is for instance preferred in starch used for industrial applications. For example, starch comprising granules with a more uniform size has more uniform gelling properties, which is an advantage in many processing procedures.

In a further embodiment the invention provides a method according to the invention for producing starch having at least one altered characteristic, wherein said modification and/or alteration further comprises a modification of starch granule morphology. Said starch granule morphology is defined as the shape or form of said starch granule. The external aspect of said shape or form is for instance made visible by light and/or Scanning Electron Microscopy (SEM). Said modification of starch granule morphology for instance comprises addition of polysaccharide on the starch granule surface. Said modification of said starch granule morphology is preferably a modification that leads to a coarse image of a starch granule compared to a starch granule of a control plant. Said coarse image for instance includes more protrusions and impressions on the surface of said starch granule. Further, said modification of starch morphology includes a more porous starch granule relative to a starch granule produced by a control plant. Said more porous starch granule is at least more porous with respect to the outer layer. A more porous starch granule is for instance advantageous as the more porous character improves a derivatization procedure of for instance industrial applications. Said derivatization procedure includes all derivatization procedures but is preferably a chemical derivatization procedure. The invention discloses that expression of amylosucrase, even without introduction of a granule-targeting sequence, affects starch granule morphology. Therefore, the invention in one embodiment provides a method for producing starch having at least one altered characteristic according to the invention, wherein said alteration comprises a modification of starch granule morphology. In another embodiment the invention provides a method for modifying a starch producing plant according to the invention, wherein said modification comprises a modification of starch granule morphology. Preferably however, the invention provides a method for modifying a starch producing plant and/or for producing starch having at least one altered characteristic according to the invention, comprising introducing in a starch producing plant a nucleic acid comprising an amylosucrase encoding sequence functionally linked to a granule-targeting sequence and further functionally linked to a promoter sequence active in a starch storage organ of said plant.

A characteristic of starch and/or a starch granule that is preferably altered is starch viscosity. Said starch viscosity is for instance measured by plotting a starch viscosity profile. Examples of starch viscosity parameters that are altered are peak viscosity, the onset temperature of pasting, and viscosity of starch paste. The invention thus in one embodiment provides a method according to the invention for producing starch having at least one altered characteristic, wherein said altered characteristic is starch viscosity. The invention in another embodiment provides starch according to the invention, wherein at least one characteristic of one starch granule is altered compared to a starch granule derived from a control plant, wherein said characteristic is starch viscosity.

The invention in one embodiment provides a cell comprising a nucleic acid and/or a fusion protein according to the invention. Further, the invention provides a plant or a part thereof comprising a cell according to the invention or produced by a method according to the invention. Said part of a plant may be any part of a plant but is preferably a starch storage organ. Therefore, the invention in a preferred embodiment provides a part of a plant according to the invention, wherein said part is a starch storage organ. In a preferred embodiment of the invention said plant is a potato plant. The invention thus in a preferred embodiment provides a plant or a part thereof according to the invention, wherein said plant is a potato plant. In a further preferred embodiment the invention provides a plant or a part thereof according to the invention wherein said part is a tuber. In a preferred embodiment of the invention said tuber is a potato. Although the invention is suitable for any starch producing plant, said plant is preferably an amylose-containing plant. Therefore, the invention in a preferred embodiment provides a plant or part thereof according to the invention, wherein said plant is an amylose-containing potato plant.

The invention provides new methods and means for producing starch. The invention therefore provides starch obtainable and/or obtained by a method according to the invention, and/or obtainable and/or obtained from a plant and/or part thereof according to the invention. In one embodiment of the invention, a characteristic of a starch granule is altered relative to a starch granule that a plant of the same species produces without introduction of an amylosucrase encoding sequence, i.e. control plant. The invention thus provides starch according to the invention, wherein at least one characteristic of one starch granule is altered compared to a starch granule derived from a control plant. A characteristic that is preferably altered is starch granule size. The invention thus provides starch according to the invention, wherein said characteristic is starch granule size. Said granule size is preferably increased compared to a control plant. The invention thus in a preferred embodiment provides starch according to the invention, wherein said starch granule size is increased. Another characteristic that is preferably altered is starch granule morphology. The invention therefore in one embodiment provides starch according to the invention, wherein further starch granule morphology is altered. The invention also provides derivatives of starch and/or starch granules according to the invention. Examples of such derivatives are processed starch, an amylose fraction of starch and a protein fraction derived from starch granules wherein at least one additional sequence encoding a protein is introduced in a starch producing plant. Furthermore, the invention provides any product comprising a plant or part thereof according to the invention and/or starch and/or starch granule according to the invention. A product according to the invention is for instance a food and/or an industrial product. Examples of industrial products are paper products, textile warp additives and adhesive compounds. Examples of food products are for instance chips, bread or cereal products. The invention thus in one embodiment provides food and/or an industrial product comprising a plant or part thereof according to the invention and/or starch according to the invention. The invention further in one embodiment provides a bacterium, preferably an *Agrobacterium,* more preferably an *Agrobacterium tumefaciens* comprising an amylosucrase encoding sequence functionally linked to a granule-targeting sequence and functionally linked to a promoter sequence active in a starch storage organ and/or comprising a fusion protein according to the invention.

The invention further provides use of all means provided by the invention. The invention thus for instance provides use of an amylosucrase encoding sequence functionally linked to a granule-targeting sequence and functionally linked to a promoter sequence active in a starch storage organ for producing starch having at least one altered characteristic. The invention also provides use of a fusion protein, a cell, a plant or part thereof, starch and/or products according to the invention.

### Examples

### Materials and methods

### Preparation of constructs

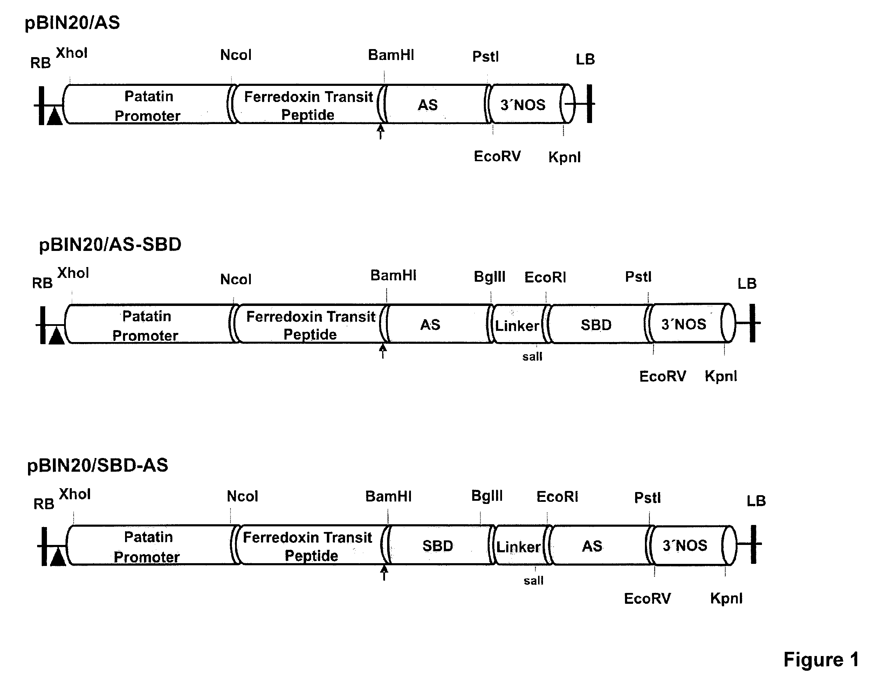

Three different constructs were made in this study, the pBIN20/AS-SBD and pBIN20/SBD-AS plasmids were used for the expression of the AS-SBD and SBD-AS fusion proteins in the amylose-containing potato plants (Kardal) respectively. The pBIN20/AS plasmid was used as a control in which amylosucrase was directed to the amyloplast without granule-targeting sequence (SBD).
The pBIN20/AS-SBD plasmid was constructed as follows. An expression cassette for the AS-SBD gene was first assembled in a pBlueScript vector from three DNA fragments: (1) the tuber-specific patatin promoter and a ferredoxin signal sequence (XhoI-BamHI), (2) an AS-SBD fragment (BamHI-PstI), and (3) the NOS terminator sequence (EcoRV-KpnI). The AS-SBD fragment (BamHI-PstI) was amplified from the pTrcHisB/AS-SBD plasmid by PCR using the following primers: 5'-CGAAAA**GGATCC**CCCGAAT-3' and 5'-TCT**CTGCAG**CGCCAAA.ACA-3', which contained BamHI and PstI sites, respectively. The pBlueScript/AS-SBD was digested with XhoI and KpnI, and the fragment (XhoI-KpnI) was inserted into the corresponding sites of the pBIN20 vector, giving plasmid pBIN20/AS-SBD (Fig. 1). The size of the expected AS-SBD fusion protein accumulated in potato tubers has a predicted molecular mass of 86,468 Da, excluding the transit peptide.
For making the pBIN20/SBD-AS plasmid, a SBD-AS fragment (BamHI-PstI) was amplified from the pTrcHisB/SBD-AS plasmid by PCR with primers 5'-TGACGAAAA**GGATCC**ATTGTC-3' and 5'-TA**CTGCAG**GCATTTGGGAA-3', which contained BamHI and PstI sites, respectively. The amplified SBD-AS fragment (BamHI-PstI) was used to replace the AS-SBD fragment in the pBlueScript/AS-SBD plasmid to generate plasmid pBlueScript/SBD-AS. The rest of the procedure was conducted in the same as described for pBIN20/AS-SBD. The resultant plasmid is referred to as pBIN20/SBD-AS (Fig. 1). The SBD-AS fusion protein accumulated in potato tubers has a predicted molecular mass of 86,110 Da, excluding the transit peptide.
For making the pBIN20/AS plasmid, an amylosucrase-encoding fragment was obtained by PCR amplification using the pGSTAS plasmid as a template with the primers 5'-CGAAAA**GGATCC**CCCGAAT-3' and 5'-TA**CTGCAG**GCATTTGGGAA-3', containing BamHI and PstI sites, respectively. The plant-expressed AS has a predicted molecular mass of 72,466 Da, excluding the transit peptide. All constructs were verified by DNA sequencing.

### Plant transformation and regeneration

The pBIN20/AS, pBIN20/AS-SBD, and pBIN20/SBD-AS plasmids were transformed into *Agrobacterium tumefaciens,* according to the three-way mating protocol described by Visser et al (1991). Internodal stem segments from the amylose-containing (Kardal) potato were used for *Agrobacterium-*mediated transfomation containing the plasmid (Visser et al, 1991). More than 50 independent shoots from each construct were harvested. Shoots were tested for root growth on a kanamycin-containing (100 mg/L) MS30 medium (Murashige and Skoog, 1962). Thirty transgenic, root-forming, shoots from each construct were multiplied and transferred to the greenhouse for tuber development. In addition, 10 untransformed controls were grown in the greenhouse. In this study obtained transformant series are referred to as KDA*xx,* KDAS*xx* and KDSA in which A, AS and SA stand for the *Amylosucrase, Amylosucrase-SBD and SBD-Amylosucrase* fusion proteins, respectively, xx represents the clone number. In this study KD-UT represents the untransformed control plant.

### Determination of AS gene transcript levels

Total RNA was prepared from frozen plant material using 5 g (fresh weight) of potato tuber material according to Kuipers *et al.* (1994). Following electrophoresis (1.5% (w/v) agarose, 15% formaldehyde (w/v)), RNA was transferred to nylon membranes Hybond-N (Amersham, Amersham, UK) overnight. Membrane were hybridized overnight at 65°C in modified church buffer containing 7% (w/v) SDS, 0.5% sodium phosphate (w/v) buffer and 1 mM EDTA, pH=7.2. The membranes were hybridized with a [³²P]-labeled amylosucrase DNA fragment (BamHI-PstI) as a probe; labeling was performed using a *redi*prime™II kit (Amersham, Amersham, England), according to the instructions of the manufacturer. Membranes were washed at 65°C once with 2 x SSC for 5 minuets and 30 minuets in 1 x SSC, each containing 0.1% (w/v) SDS.

### Isolation of tuber starch

All tubers derived from five plants of each greenhouse-grown clone were combined, and their peels were removed in an *IMC Peeler* (Spangenberg, The Netherlands). The peeled tubers were homogenized in a *Sanamat Rotor* (Spangenberg), and filtered through a sieve to remove particulate material. The resulting homogenate was allowed to settle for at least 20 min to over night at 4 °C, and the tuber juice was collected for later use, and stored at -20 °C. The starch sediment was washed three times with water, and finally air-dried at room temperature (Ji *et al.,* 2003).

### Determination of AS-SBD and SBD-AS protein content of transgenic starches by Western dot blot analysis

A 12.5% sodium dodecyl sulfate-polyacrylamide (SDS-PAGE) gel (50 × 50 × 3 mm) with 9, equally-spaced, holes (Ø=9 mm) was placed in contact with a similarly-sized Hybond ECL nitrocellulose membrane (Amersham Pharmacia Biotech, UK). Twenty mg of (transgenic) starch was boiled for 5 min with 200 µL of a 2 × SDS sample buffer (Murashige and Skoog, 1962). After cooling to room temperature, the starch gel was transferred into one of the holes. The proteins from transgenic starch gels were blotted to the membrane using a PhastSystem (Pharmacia, Uppsala, Sweden; 20 V, 25 mA, 15°C, 45 min) (Ji *et al.,* 2003). The AS-SBD and SBD-AS fusion proteins were identified with anti-SBD antibodies according to the method described by Ji *et al.* (2003).

### Determination of AS-SBD and SBD-AS protein content in tuber juice by Western dot blot analysis

The AS-SBD and SBD-AS fusion proteins in the soluble fraction were determined as follows. 500 µL of tuber juice was freeze-dried. The dried material was dissolved in 200 µL of 2 × SDS sample buffer, boiled for 5 min in the presence of 20 mg starch from the control samples. The forth-coming starch gel was applied to one of the holes in the SDS-PAGE gel. The rest of the procedure was conducted in the same way as described for the granule-bound proteins.

### SDS-PAGE and Immunoblotting analysis of granule bound fusion proteins

Western blotting of the starch granule-bound proteins was carried out as described by Nazarian *et al* (2006b), using sodium dodecyl sulfate-polyacrylamide gel electrophoresis (SDS-PAGE). Fifty milligrams of dry starch sample from KDAS2 and KDSA2 were solubilized in 1 mL SDS sample buffer (final concentrations: 1M Tris-HCL, pH 6.8, 2% SDS (w/v), 10% glycerol (w/v), 3% B-mercaptoethanol (w/v)) and boiled for 2 min prior to analysis on a gel. 25 microliters were loaded onto lanes of 12% polyacrylamide gel (145mm x95mm x3mm, Bio Rad, UK). Proteins were transferred onto a Hybond ECL nitrocellulose membrane (Amersham Pharmacia Biotech, Amersham, UK) and imunoblotted using anti SBD as described in previous sections.

### Analysis of physico-chemical properties of starch granules

Average granule size and granule size distribution of the starches were determined with a Coulter Multisizer II, equipped with an orifice tube of 100 µm (Beckman-Coulter, High Wycombe, UK). Approximately 10 mg of starch was dispersed in 160 mL of Isoton II. The granule size distribution was recorded by counting approximately 50,500 (±500) particles. The coincidence (the frequency of two granules entering the tube at the same time, and consequently being counted as one) was set at 10%.
Starch granule morphology was investigated by light microscopy (LM, Axiophot, Oberkochen, Germany) and Scanning Electron Microscopy (SEM, JEOL 6300F, Tokyo, Japan). For LM, starch granules were stained with a 20x diluted Lugol's solution (1% I₂/KI). For SEM, dried starch granules were mounted onto brass holders with double-sided sticky carbon tape (EMS, Washington, U.S.A.), sputter coated with cold, (Edwards S150B, Crawley, England) and subsequently transferred into a FESEM (JEOL JSM-6300F, Tokyo, Japan). The samples were analyzed and recorded with SE detection at 5 kV and a working distance of 15 mm. All images were recorded digitally (Orion, 6 E.L.I. sprl, Belgium) at a scan rate of 100 seconds (full frame) at the size of 2855 x 2154, 8 bit.
The apparent amylose content was determined according to the method described by Hovenkamp-Hermelink *et al.* (1989). The temperature at which starch granules start to gelatinize was determined by differential scanning calorimetry (DSC) using a Perkin-Elmer Pyris 1 (Perkin-Elmer, Vlaardingen, The Netherlands), equipped with a Neslab RTE-140 glyco-cooler (Ji *et al.,* 2003).
Starch viscosity parameters were measured from a 5% (w/v) and 2% (w/v) starch suspension by employing a Thermo Haake rheoscope (Thermo Haake, Karlsruhe, Germany). The machine was equipped with parallel plate geometry (type C70/1 Ti) and the gap size was 0.1 mm. Oscillating shear deformation was recorded at a frequency of 1 Hz. The pasting profile of starch suspensions (5% and 2%) was obtained by subjecting the suspensions from 40° C to 90° C at 2° C per min, 15 min at 90° C and cooling down to 20° C at 2° C/min, a constant temperature of 20° C for 15 min. Starch properties such as T_{g} (start gelatinization temperature, Tₚ (peak temperature) and the corresponding temperature were measured. Subsequently, to check the linearity of the measurement in a linear viscoelastic region, the sample was subjected to an amplitude sweep region, in which the amplitudes of stress and strain are proportional to each other.

### Determination of starch content

Approximately 50 mg of potato tuber material was dissolved in 0.5 mL of 25% HCl and 2 mL of dimethylsulfoxid (DMSO) for 1 h at 60 °C. After incubation the mixture was neutralized with 5 M NaOH and diluted in 0.1 M citrate buffer (pH 4.6) to a final volume of 10 mL. 20 µL of the hydrolyzed starch sample was determined enzymatically using a test kit (Boehringer-Mannheim, Mannheim, Germany), according to the instructions of the manufacturer.

### Chain length distribution of transgenic starch

To test the chain length distribution, 5 mg starch from transgenic potatoes was suspended in 250 µl of DMSO and gelatinized for 10 min at boiling temperature. After cooling down to the ambient temperature, 700 µl of 50 mM NaAc buffer PH 4.0 was added. A sufficient amount of isoamylose (Hayashibara Biochemical laboratories, Okayama, Japan; 59,000 U/mg protein) to debranch the starch polymers completely was added to the mixture, which was incubated for 2 h at 40 °C. After inactivation of the enzyme for 10 min at boiling temperature,1 ml of 25% DMSO was added. For High-Performance Size-Exclusion Chromatography (HPSEC), the samples were used as such (HPSEC). The HPSEC was performed on a p680HPLC pump system (Dionex, Sunnyvale, Cal. USA) equipped with three TSKgel SWXL columns in series (one G3000 and two G2000: 300mmx7.5mm; Montgomeryville, USA) in combination with a TSKgel SWXl guard column (40mmx6mm) at 35°C. Aliquots of 100 µl were injected using a dionex ASI-100 Automated Sample Injector, and subsequently eluted with 10 mM NaAc buffer (ph 5.0) at a flow rate of 0.35 ml/min (3h run). The effluent was monitored using a RID-6A refractometer (Shimadzu, the Netherlands). The system was calibrated using dextran standards (10,40,70,500 kDa; Pharmacia). Dionex chromelon software version 6.50 SP4 Build 1000 was used for controlling the HPLC system and data processing.
HPAEC was used to obtain a better separation of the smaller amylopectin side chains (in the range of 245 glucose residues). HPAEC was performed on a GP40 gradient pump system (Dionex) equipped with a carboPac PA 100 column (4mmx250mm; Dionex) at 35 °C. The flow rate was 1.0ml/min and 20 µl samples were injected with Dionex AS3500 automated sampler. Two eluents were used, eluent A (100mm NaOH) and eluent B (1 M NaAc in 100 mm NaOH), for mixing the following gradient: 0→5 min,100% eluent B(rinsing phase);5→20 min, 100% eluent A (conditioning phase);20→25 min, linear gradient from 0→20% eluent B (100→80% eluent A);25→50 min, linear gradient from 20→35% eluent B (80→65% eluent A);50→55 min, linear gradient from 35→50% eluent B(65→50% eluent A);55→60min , 50% eluent B (50% eluent A). The sample was injected at 20 min. The eluent was monitored by ED40 electrochemical detector in the pulsed amperometric mode (Dionex).

### Incubation of transgenic starch with sucrose

A 10 mg portion of (transgenic) tuber starch was incubated in 1 mL of 50 mM Tris-HCl (pH 7.0) containing 10 mM sucrose for 48 and 96 at 40 °C with continuous mixing. After incubation, the samples were centrifuged (7,500 × g*,* 10 min), the supernatants were collected, and concentrations of sucrose, glucose and fructose were measured by HPAEC according to Nazarian *et al.,* (2006a). The starch sediment was washed three times with water, and air-dried at room temperature. After debranching with iso-amylose, the starch was examined for different chain length distribution by both HPAEC and GPC.

### Determination of digestibility of starch granules by α-amylase treatment

Twenty mg of (transgenic) starch was suspensed in 1 mL of 50 mM NaOAc buffer (pH 6.9) and treated with 5 u of α-amylose (porcine pancreas, Sigma, The Netherlands) for 4 h at 25 °C. Sample without α-amylose addition served as a control. After incubation, the samples were centrifuged (7,500 × *g*, 10 min). The supernatants were analyzed by HPAEC for the release of glucose and maltooligosaccharides (MOS).

### Results

### Amylosucrase is accumulated in the transgenic starch granules and juice fractions

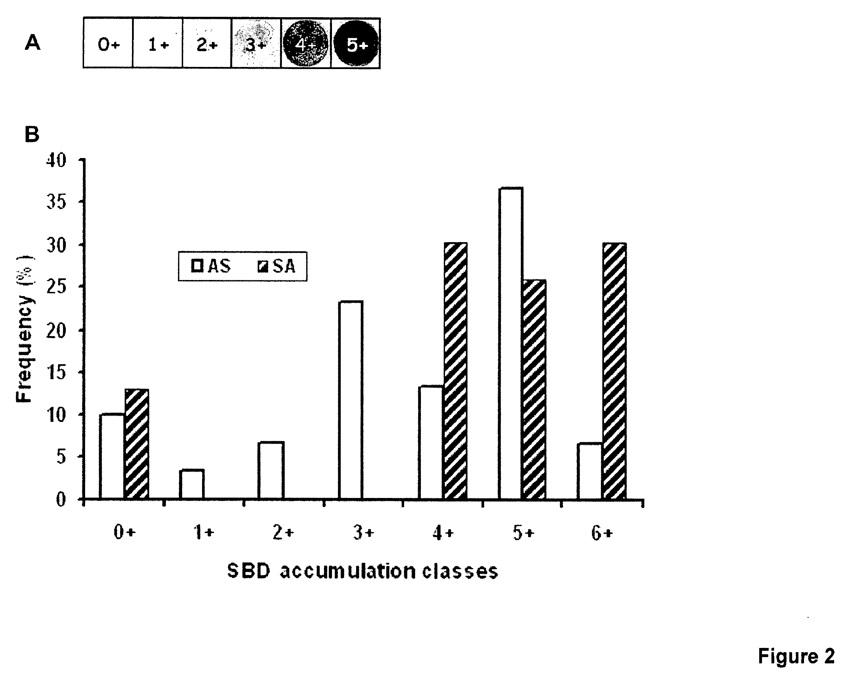

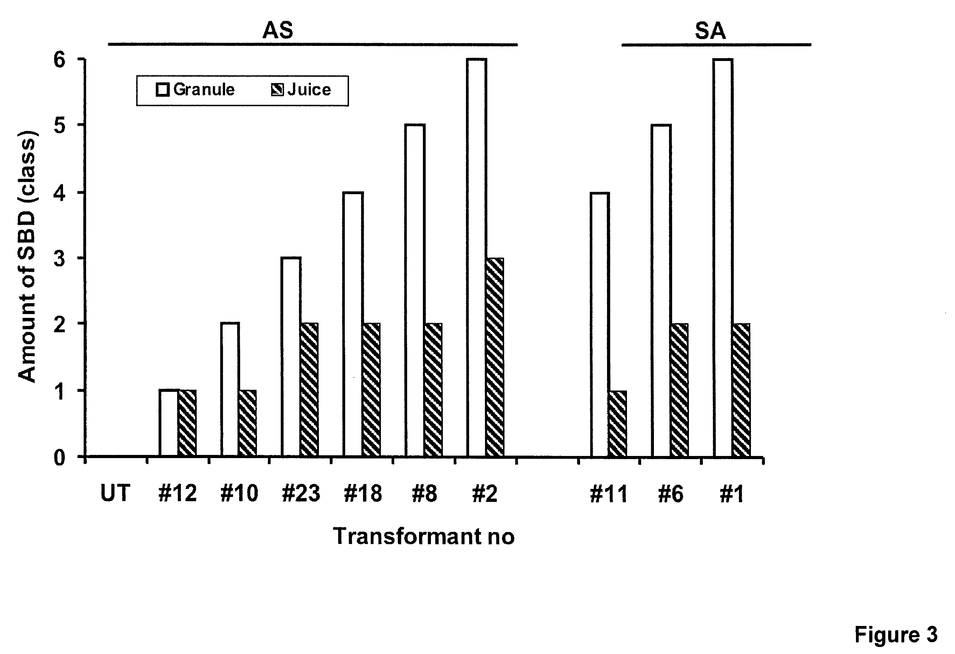

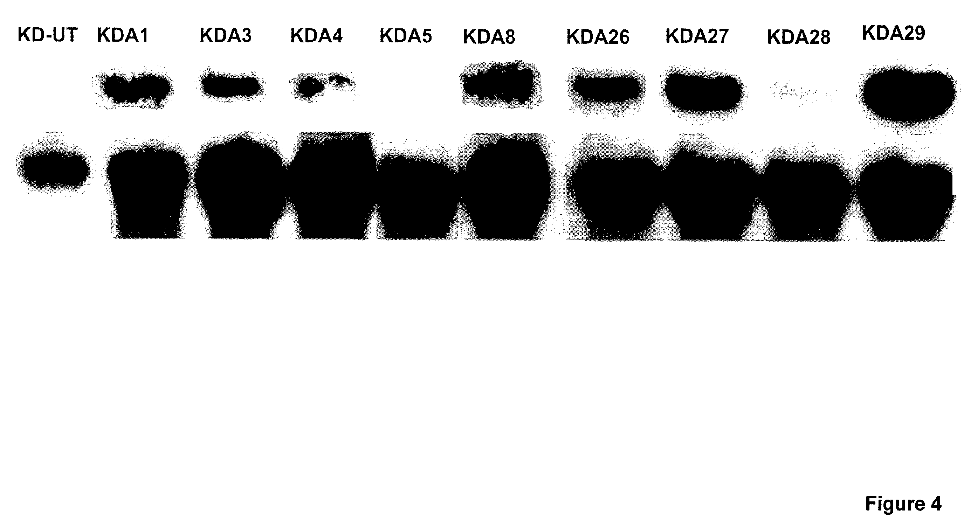

In this study three different open reading frames (ORFs) consisting of the mature *Amylosucrase* from *Neisseria polysaccharea* and a starch binding domain (SBD) encoding region of *cyclodextrin glycosyltransferase* from *Bacillus circulans* were made in the pBIN20 binary vector. A 950 base pairs segment of the potato patatin promoter (Wenzler *et al.,* 1989) and the choloroplastic ferredoxin signal peptide (Pilon *et al.,* 1995) were placed 5' to the ORFs to induce their tuber specific expression and amyloplast entry respectively (Fig. 1). Upon growth in the greenhouse, plants looked normal with respect to their morphology and phenotype compared to the control plants.
Fusion protein expression was investigated using Western dot blot analysis in *AS*xx and SAxx series of transgenic potato plants, using anti SBD serum (Ji *et al.,* 2003). The scoring method was used according to SBD2 expression in mutant *amf* series, using numbers from 0+ to 6+ (Ji *et al.,* 2004). Transgenic potato plants were divided into 6 different categories with 0+ showing no protein and 6+ with the highest level of fusion protein accumulation (Fig. 2A). Screening of fusion protein transformants based on Western dot blot analyses revealed that transformants with higher amounts of fusion protein accumulation are more than those with less fusion protein accumulation (Fig. 2B). From the AS-SBD transformants 17 out of 30 (56.7%) and from the SBD-AS transformants 20 out of 23 (86.9%) showed an intensity falling in classes 4+ through 6+ class which was significant (X²=12.56, P<0.001). Moreover the location of the granule targeting sequence (SBD) seemed to influence the accumulation level of the fusion proteins, i.e. SBD-AS fusion protein was accumulated in significant (X²=9.36, P<0.001) larger amounts than AS-SBD fusion because almost all of the SBD-AS transformants (except 3 in 0+ class) showed an intensity comparable to 4+ or higher (Fig. 2B) while no transformant was detected with lower protein accumulation.
Western dot blot analyses for a number of the selected transformants on the soluble fraction part of the potato juices were done. Protein detection results for the selected transformants in both starch granules and their corresponding juice fractions are summarized in Figure 3. It can be seen from the graph that fusion proteins can be found from 1+ class onwards in the juices. There is some correlation between fusion protein accumulation level in granules and their corresponding juice fraction in this case (Fig. 3).
In order to determine expression level of introduced amylosucrase alone in potato tubers and screening of transformants, a Northern blot analysis with total RNA extracted from tuber materials was carried out (because of lack of amylosucrase antibody). Three different categories (none, low and high) were recognized based on mRNA transcript levels (Fig. 4, upper panel), using 28 rRNA ribosomal probe as an internal control (Fig. 4, lower panel). Sixteen, twelve, sixteen and fifty six percent of transformants were classified as high (H), medium (M), low (L) and none (N) expressers.

### Transgenic potato plants contain amylosucrase in their starch fraction

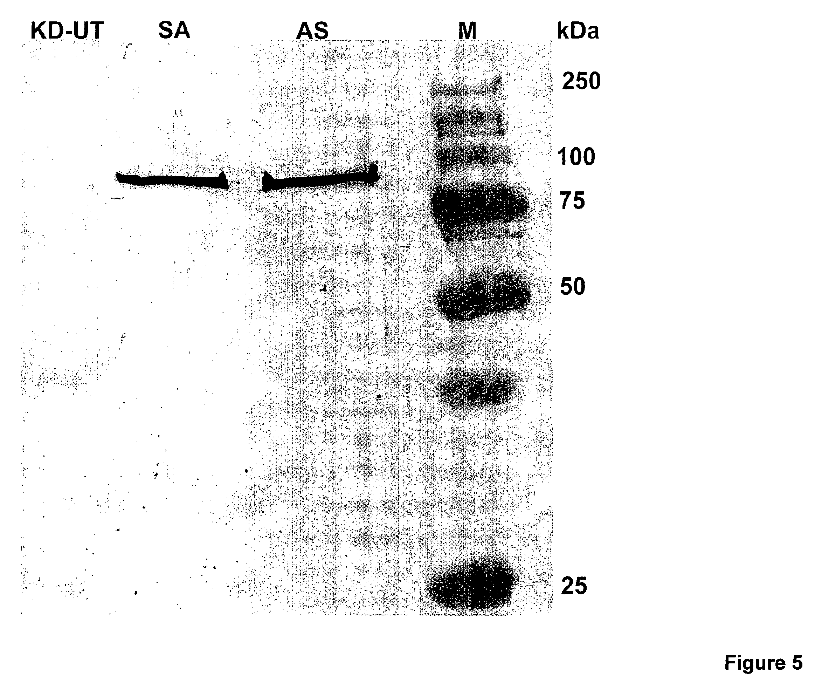

In order to examine the presence of fusion proteins accumulated in starch granules of potato plants, the alien starch granule-bounded proteins of one representative of each fusion protein were analysed by SDS-PAGE followed by immunoblotting detection using anti SBD (Fig. 5). Proteins of about 85 kDa were recognized in which nicely corresponds to the predicted molecular mass of 86.1 kDa and 86.5 kDa for SBD-AS and AS-SBD fusion proteins respectively. No band was found with starches from control plants (Fig. 5).These results clearly showed that amylosucrase has been successfully targeted to the starch granules during starch biosynthesis.

### Amylosucrase expression leads to altered starch granules

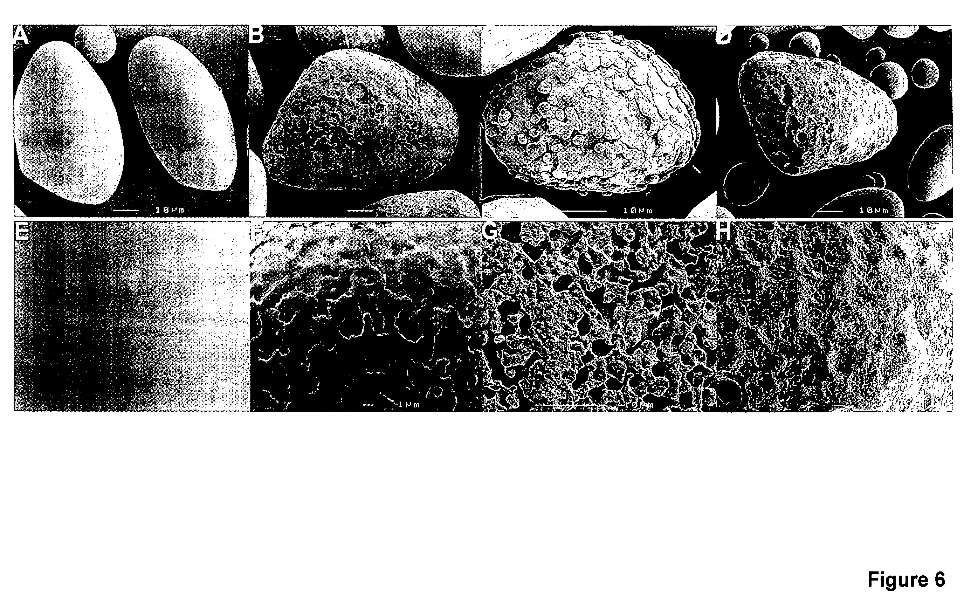

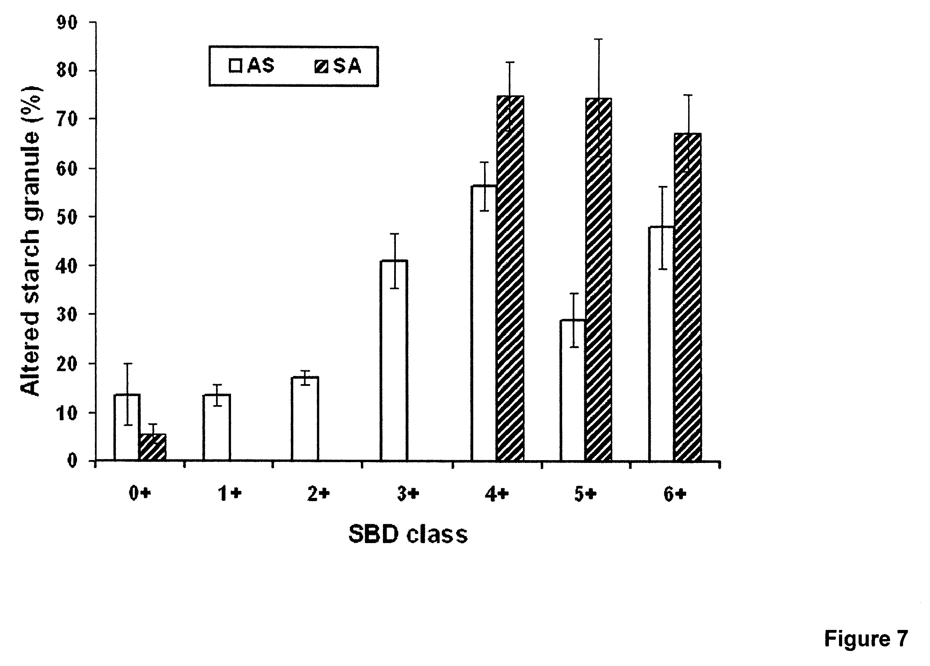

Light microscopy as well as Scanning Electron Microscopy (SEM) imaging for the transgenic potato starches revealed that starch granule morphology is severely affected. Expression of fusion proteins resulted in different granule morphology alterations. The appearance of altered granules was different between the C- and N- terminal heterologous SBD fusion proteins (Fig. 6 compare C and D). Surprisingly, expression of amylosucrase alone affected the starch granule morphology. However the appearance of the altered starch granules was different than the fusion proteins expressers (Fig. 6 compare B to C and D). The starch granule surface in all three different series of transformants was different, SA transformants had a thicker layer of extra polysaccharide in comparison to the other two. The surface appearance of the AS and amylosucrase alone expressers seems to be the same with respect to the extra polysaccharide layer, however the amylosucrase expressers have tiny protrusions (Fig. 6, compare F with H). In order to quantify the number of altered starch granules in different classes of fusion proteins (Fig. 7) as well as different amylosucrase expressers, a population of two hundred granules from different transformants were counted. Figure 7 highlights two main messages. First, there seems to be a correlation between the protein level and altered starch granule percentage and second, the N- terminal SBD (AS-SBD) seems to have somewhat more impact on starch granules. In the amylosucrase transformants a 15% (±1.2) and 6% (±2.1) of altered granules were found for the high (H) and intermedium (M) amylosucrase expressers respectively.

### Expression of AS with SBD leads to increase in granule size

Starch granule size is usually expressed as a distribution of sizes which is characteristic in the particular starch type. Although expression of amylosucrase alone changed the granule morphology (Fig. 6 F), it did not result to granule size alteration (Table. 1). On the contrary, expression of both AS and SA fusion proteins significantly increased the granule size two folds from 20.8 µm average to 47.4 (Fig. 9). In AS and SA series, 83% (25 out of 30) and 70% (16 out of 23) showed a granule size bigger than the control plants (20.8 µm). With respect to the SBD location, the best transformants in both series demonstrated the same fold increase in granule size (Fig. 9).
In order to compare mean granule size between AS and SA series, one way ANOVA (analysis of variance) was done (Table 1). With respect to the SBD location, there was no significant difference between AS and SA transformant series in starch granule size (F=1.09, P=0.343). A correlation analysis showed that there is a positive correlation between the SBD level (class) and granule size in both series. However this correlation was much stronger in AS transformants (p<0.001) than SA transformants (p=0.078).

### Starches from transgenic plants with bigger granules have a different viscosity behaviour

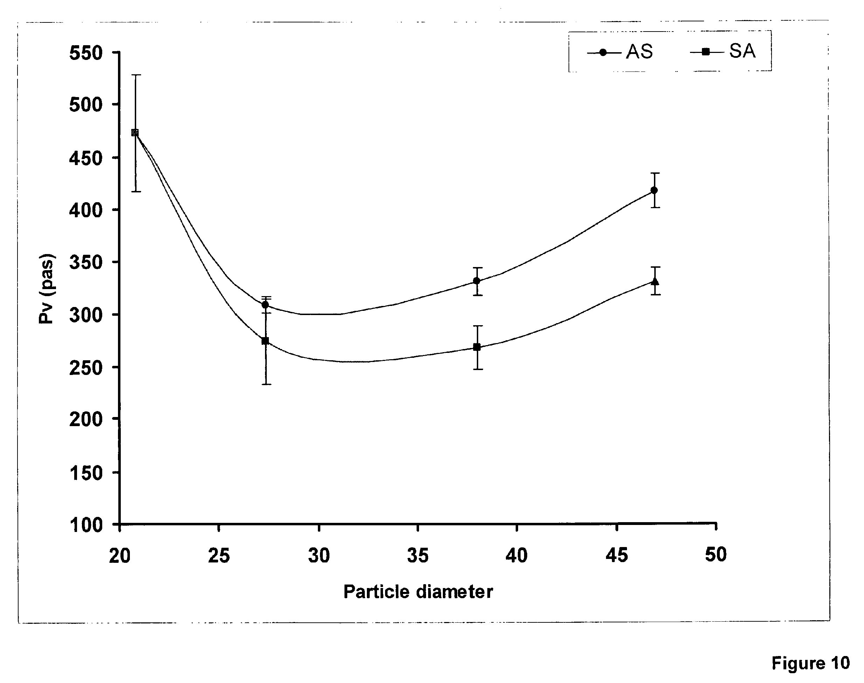

We have so far demonstrated the impact of amylosucrase in the form of fusion proteins on starch granule size. Our experiments have also pointed out that granule size does have a correlation with the amount of the protein accumulated in the granules. The next question is what would be the consequence (s) of having bigger granules on starch properties? Starch viscometric analyses showed that transgenic starches with bigger granules had a different behaviour from the control starches as well as small granules. As shown in the Table. 1, the T onset is somewhat higher in starches with bigger granules. This however, does not reflect a correlation as such in protein level. Starch viscosity profile as it is heated and cooled to the initial temperature (20°C) under a constant stirring manner was different in transformants.
Starch peak viscosity (the highest viscosity reached during the gelatinization of starch usually corresponding to the point where all the granules are swollen to their maximum level) was different in transgenic plants. In both AS and SA series the peak viscosity decreases when the particle size increases to almost 30 µm but it increases later on (Fig. 10). Viscosity of starch pastes during cooling process was also affected by granule size.

### Some starch characteristics are not significantly deviated

Analysis of starch properties such as apparent amylose content (%AM) and T onset were also done. No significant change (s) was observed. Starch yield of transformants were the same as control plants. Chain length distribution profiles after isoamylase complete debranching was comparable to that of the untransformed plants (data not shown). Post harvest experiments with transgenic starches to get the granule bound amylosucrase activated in the presence of different sucrose concentrations did not show any amylose like polymer synthesis or did not show any change in HPAEC profiles, indicating sucrose splitting and glucose consumption (data not shown).

| **Table 1**. An overview of different starch properties is shown. Apparent amylose content, median of granule size and T onset are shown. All data are average of three independent measurements (±SD). | | | | |
|---|---|---|---|---|
| **Clone** | **Wb** | **d50** | **%AM** | **T onset** |
| KD-UT | Nd | 20.8 | 20.0 (±0.1) | 65.9 (±0.1) |
| AS2 | 6+ | 38.0** | 21.6 (±0.4) | 67.2 (±0.4) |
| AS5 | 5+ | 41.0** | 20.5 (±0.5) | 67.1(±0.2) |
| AS6 | 6+ | 47.4** | 19.9 (±0.3) | 66.7 (±0.1) |
| AS8 | 5+ | 37.3** | 21.1 (±0.1) | 67.2 (±0.4) |
| AS30 | 6+ | 36.1** | 20.7 (±0.2) | 66.2 (±0.1) |
| AS14 | 4+ | 27.4* | 21.1 (±0.4) | 64.7 (±0.2) |
| SA1 | 6+ | 37.4** | 19.9 (±0.1) | 66.8 (±0.1) |
| SA2 | 6+ | 40.9^{**} | 20.0 (±0.1) | 67.1(±0.2) |
| SA7 | 5+ | 31.7^{**} | 19.3 (±0.1) | 66.9 (±0.4) |
| SA15 | 4+ | 21.2^{ns} | 18.6 (±0.3) | 64.2 (±0.4) |
| SA18 | 0+ | 19.9^{ns} | 19.2 (±0.2) | 65.2 (±0.1) |

| | | | | |
|---|---|---|---|---|
| Wb=Western blot, d50= Median granule size, AM= apparent amylose content, Nd=not detected, *, ** significant at α=0.05 and α=0.01 respectively, ns=not significant. | | | | |

**Table 2. Tissue specific promoters (seed and tubers)**

| **Promoter** | **source** | **Reference** |
|---|---|---|
| **GBSSI** | **Potato** | **Van der leij *et al,* 1991** |
| **Patatin** | **Potato** | **Wenzler *et al,* 1989** |
| **Sbe I** | **Rice** | **Kawasaki *et al,* 1993** |
| **Prolamin NPR33** | **Rice** | **Wu *et al,* 1998** |
| **Zein** | **Maize** | **Penderson *et al,* 1982** |
| **Lectin *(psI)*** | **Pea** | **Gatehouse *et al,* 1987** |
| **USP** | **Broad bean** | **Baümlein *et al*, 1991** |
| **SBP** | **Broad bean** | **Heim *et al,* 2001** |
| **Legumin B4** | **Broad bean** | **Baümlein *et* al, 1986** |
| **Phaseolin** | **Bean** | **Slightom *et al,* 1983** |

### References

1. Baümlein H, Wobus U, Pustell J, Kafatos F (1986) The legumingene family: structure of a B-type gene of Vicia faba and a possible legumin gene specific regulatory element. Nucleic Acids Res 14: 2707-2720.
2. Baümlein H, Boerjan W, Nagy I, Bassuner R, Van Montagu M, Inze D, Wobus U (1991) A novel seed protein gene from Vicia faba is developmentally regulated in transgenic tobacco and Arabidopsis plants. Mol Gen Genet 225: 459-67.
3. De Montalk GP, Remaud-Simeon M, Willemot RM, Planchot V, Monsan P (1999) Sequence analysis of the gene encoding amylosucrase from Neisseria polysaccharea and characterization of the recombinant enzyme. J Bacteriol 181: 375-81.
4. Gatehouse JA, Bown D, Evans IM, Gatehouse LN, Jobes D, Preston P, Croy RR (1987) Sequence of the seed lectin gene from pea (Pisum sativum L.). Nucleic Acids Res 15: 7642.
5. Heim U, Wang Q, Kurz T (2001) Expression patterns and subcellular localization of a 52 kDa sucrose-binding protein homologue of Vicia faba (VfSBPL) suggest different functions during development. Plant Mol Biol 7: 461-474.
6. Hovenkamp-Hermelink JHM, De Vries JN, Adamse P, Jacobsen E, Witholt B, Feenstra WJ (1989) Rapid estimation of the amylose/amylopectin ratio in small amounts of tuber and leaf tissue of the potato potato. Res 32: 241-246.
7. Ji Q, Oomen RJFJ, Vincken JP, Bolam DN, Gilbert HJ, Suurs LCJM, Visser RGF, (2004) Reduction of starch granule size by expression of an engineered tandem starch-binding domain in potato plants. Plant Biotechnology Journal 2: 251.
8. Ji Q, Vincken JP, Suurs LC, Visser RG (2003) Microbial starch-binding domains as a tool for targeting proteins to granules during starch biosynthesis. Plant Mol Biol 51: 789-801.
9. Kawasaki T, Mizuno K, Baba T, Shimada H (1993) Molecular analysis of the gene encoding a rice starch branching enzyme. Mol Gen Genet 237: 10-6.
10. Lawson CL, van Montfort R, Strokopytov B, Rozeboom HJ, Kalk KH, de Vries GE, Penninga D, Dijkhuizen L, Dijkstra BW (1994) Nucleotide sequence and X-ray structure of cyclodextrin glycosyltransferase from Bacillus circulans strain 251 in a maltose-dependent crystal form. J Mol Biol 236: 590-600.
11. Murashige T, Skoog F (1962) A revised medium for rapid growth and bioassay with tobacco tissue culture. Physiol. plant 15: 473-497.
12. Nazarian Firouzabadi F, Kok-Jacon GA, Vincken J-P, Ji Q, Suurs LCJM, Visser RGF (2006a) Fusion proteins comprising of the catalytic domain of mutansucrase and starch-binding domain can alter the morphology of amylose-free potato starch granules during biosynthesis. Transgenic Res (to be published).
13. Nazarian Firouzabadi F, Vincken J-P, Ji Q, Suurs LCJM, Buléon A, Visser RGF (2006b) Accumulation of multiple-repeat starch-binding domains (SBD2-SBD5) does not reduce amylose content of potato starch granules. Planta (to be published).
14. Pedersen K, Devereux J, Wilson DR, Sheldon E, Larkins BA (1982) Cloning and sequence analysis reveal structural variation among related zein genes in maize. Cell 29: 1015-26.
15. Pilon M, Wienk H, Sips W, de Swaa fM, Talboom I, van 't Hof R, de Korte-Kool G, Demel R, Weisbeek P, de Kruijff B (1995) Functional domains of the ferredoxin transit sequence involved in chloroplast import. J. Biol. Chem 270.
16. Sligthom J, Sun S, Hall T (1983) Complete nucleotide sequence of a French bean storage protein gene: phaseolin. Proc Natl Acad Sci U S A 80: 1897-1901.
17. van der Leij FR, Visser RG, Ponstein AS, Jacobsen E, Feenstra WJ (1991) Sequence of the structural gene for granule-bound starch synthase of potato (Solanum tuberosum L.) and evidence for a single point deletion in the amf allele. Mol Gen Genet 228: 240-8.
18. Visser RG, Somhorst I, Kuipers GJ, Ruys NJ, Feenstra WJ, Jacobsen E (1991) Inhibition of the expression of the gene for granule-bound starch synthase in potato by antisense constructs. Mol Gen Genet 225: 289-96.
19.Wenzler H, Mignery G, Fisher L, Park W (1989) Sucrose-regulated expression of a chimeric potato tuber gene in leaves of transgenic tobacco plants. Plant Mol Biol 13: 347-54.
20. Wu C-Y, Adachi T, Hatano T, Washida H, Suzuki A, Takaiwa F (1998) Promoters of rice seed storage protein genes direct endosperm-specific gene expression in transgenic rice. Plant and cell physiology 39: 885-889.

### Legend to the figures

**Figure 1**. Plasmid maps of three binary transformation vectors used in this study. Each construct carries the potato patatin promoter and choloroplastic ferredoxin signal peptide for tuber expression and amyloplast targeting respectively. SBD and AS stand for Starch binding domain and amylosucrase genes respectively. ▲ and ↑ signs represent kanamycin resistance gene as a selectable marker and cleavage site of the transit peptide.

**Figure 2.** (**B**) Relationship between SBD accumulation levels of the fusion proteins is illustrated. (**A**)Top panel shows 6 different categories which based on them different classes were defined (Ji *et al.,* 2003).

**Figure 3**. Accumulation of the amylosucrase fusion proteins (AS & SA) in starch as well as their juice fractions.

**Figure 4.** Primary screening and transcript expression pattern analysis of amylosucrase gene in transgenic potato plants. The picture shows the differential expression level of amylosucrase in different transformants in comparison to the KD-UT. KDA29 and KDA27 (high expressers), KDA26 and KDA8 (intermediate expressers), KDA1, KDA3, KDA4 and KDA28 (low expressers), KDA5 (none expresser)

**Figure 5.** SDS-PAGE/Immunoblot analyses of starch granule-bound proteins from KDAS2 and KDSA2 transformants. Proteins were isolated from the starch granules by boiling of transgenic starches with SDS-containing buffer. Migration of marker proteins (**M**) is shown to the right. AS and SA represent Amylosucrase-SBD and SBD-Amylosucrase fusion proteins respectively.

**Figure 6.** SEM analyses of starch granules from KD-UT (A, E and I) in comparison to that of the different selected transformants at different magnifications. KDA29 (H) (B, F and J), KDAS5 (6+) (C, G and K) and KDSA1 (6+) (D, H and L)

**Figure 7.** Percentage of granules with altered morphology for the various classes of SBD-containing fusion proteins. A population of 100 starch granules in duplicates was counted. Bars indicate (mean ±SD).

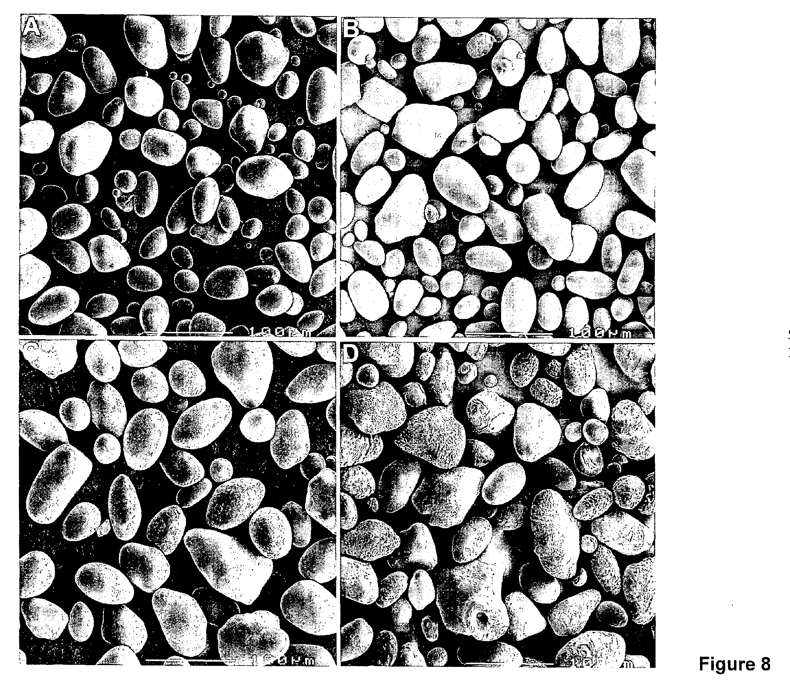

**Figure 8.** Scanning electron microscopy of control and one representative of each series of transformants are shown.

**Figure 9.** Particle size distribution of KD-UT, KDA29, KDAS5 and KDSA2 transformant starches are shown in an overlay graph.

**Figure 10.** Starch peak viscosity is shown along with particle granule size of different transformants. Each point is an average of two individual measurements.

**Figure 11.** Alignment of A.A sequences of different SBDs.

**Figure 12.** Amylosucrase alignment. N stands for Neisseria, C for Caulobacter, X for Xanthomonas.
**Figure 12a** Section 1-3
**Figure 12b** Section 4-6
**Figure 12c** Section 7-9

## Claims

1. A method for modifying a starch producing plant, comprising introducing a nucleic acid comprising an amylosucrase encoding sequence functionally linked to a granule-targeting sequence and functionally linked to a promoter sequence active in a starch storage organ of said plant into suitable plant material of said plant.

2. A method for producing starch having at least one altered characteristic, comprising introducing a nucleic acid comprising an amylosucrase encoding sequence functionally linked to a granule-targeting sequence and functionally linked to a promoter sequence active in a starch storage organ of a plant into suitable plant material of said plant and producing a plant from said material, further comprising allowing said plant to grow and harvesting starch from said plant.

3. A method according to any one of claims 1 or 2, wherein said granule-targeting sequence comprises a starch binding domain (SBD) encoding sequence.

4. A method according to any one of claims 1-3, wherein said nucleic acid is further functionally linked to an amyloplast-targeting sequence.

5. A method according to claim 4, wherein said amyloplast-targeting sequence comprises a transit peptide encoding sequence.

6. A method according to any one of claims 1-5, wherein said nucleic acid encodes a fusion protein.

7. A method according to any one of claims 1-6, wherein said amylosucrase encoding sequence encodes an amylosucrase that is at least 70% homologous to the sequence of figure 12.

8. A method according to any one of claims 1-7, wherein said SBD encoding sequence encodes an SBD that is at least 70% homologous to the sequence of figure 11.

9. A method according to any one of claims 1-8, wherein said modification or alteration comprises an increase in starch granule size.

10. A method according to any one of claims 1-9, wherein said modification or alteration comprises a shift in a distribution of starch granule size produced by said plant towards larger granules.

11. A method according to any one of claims 9 or 10, wherein said modification or alteration further comprises a modification of starch granule morphology.

12. A nucleic acid comprising an amylosucrase encoding sequence, a starch binding domain (SBD) encoding sequence and a promoter sequence.

13. A nucleic acid according to claim 12, wherein said promoter is a tuber specific promoter.

14. A fusion protein comprising amylosucrase and a starch binding domain (SBD).

15. A fusion protein according to claim 14, further comprising an amyloplast transit peptide.

16. A fusion protein according to any one of claims 14-15, wherein said starch binding domain comprises a starch binding domain of a cyclodextrin glycosyltransferase.

17. A fusion protein according to any one of claims 14-16, wherein said starch binding domain is at least 70% homologous to the SBD sequence of figure 11.

18. A cell comprising a nucleic acid according to any one of claims 12 or 13 and/or comprising a fusion protein according to any one of claims 14-17.

19. A plant or a part thereof comprising a cell according to claim 18 or produced by a method according to claim 1.

20. A part of a plant according to claim 19, wherein said part is a starch storage organ.

21. A plant or a part thereof according to claim 20, wherein said plant is a potato plant.

22. A plant or a part thereof according to any one of claims 19-21 wherein said part is a tuber.

23. A plant or part thereof according to any one of claims 19-22, wherein said plant is an amylose-containing potato plant.

24. Starch obtainable by a method according to any one of claims 2-11, and/or obtainable from a plant and/or part thereof according to any one of claims 19-23.

25. Starch according to claim 24, wherein at least one characteristic of one starch granule is altered compared to a starch granule derived from a control plant.

26. Starch according to claim 25, wherein said characteristic is starch granule size.

27. Starch according to claim 26, wherein said starch granule size is increased.

28. Starch according to any one of claims 26 or 27, wherein further starch granule morphology is altered.

29. Food and/or an industrial product comprising a plant or part thereof according to any one of claims 19-23 and/or starch according to any one of claims 24-28.
